# EUROPEAN PATENT APPLICATION

(11) **EP 2 628 793 A1**
(43) Date of publication of application: **21.08.2013**
(21) Application number: 12001091.3
(22) Date of filing: 17.02.2012
(51) Int. Cl.: C12N 1/21, C12R 1/19, C07K 14/47, A61K 35/74

(54) **Recombinant escherichia coli strains**

(71) Applicant: PHARMA-ZENTRALE GMBH, 58313 Herdecke (DE)
(72) Inventor: Ölschläger, Tobias, 97318 Kitzingen (DE); Seo, Ean Jeong, 55118 Mainz (DE); Wehkamp, Jan, 70372 Stuttgart (DE); Stange, Eduard F., 70469 Stuttgart (DE); Sonnenborn, Ulrich, 44799 Bochum (DE); Malinka, Jürgen, 59379 Selm-Bork (DE); Proppert, Hans, 58095 Hagen (DE)
(74) Representative: Kossak, Sabine

(57) **Abstract**

The present invention is directed to a recombinant *E. coli* Nissle 1917 (EcN) cell transformed with a nucleic acid coding for a defensin protein or a derivative thereof. The invention is further directed to a pharmaceutical composition comprising this cell and a pharmaceutically acceptable carrier as well as a method of producing a recombinant *E. coli* Nissle 1917 cell and its use in the treatment of Crohn's disease.

SEQUENCE LISTING

<110>  Julius-Maximilians-Universität Würzburg

<120>  RECOMBINANT E. COLI STRAINS

<130>  Pt-2011-00123

<160>  13

<170>  PatentIn version 3.5

<210>  1
<211>  195
<212>  DNA
<213>  artificial

<220>
<223>  HBD2 cDNA

<400>  1
atgagggtct tgtatctcct cttctcgttc ctcttcatat tcctgatgcc tcttccaggt      60

gtttttggtg gtataggcga tcctgttacc tgccttaaga gtggagccat atgtcatcca     120

gtcttttgcc ctagaaggta taaacaaatt ggcacctgtg gtctccctgg aacaaaatgc     180

tgcaaaaagc catga                                                      195


<210>  2
<211>  201
<212>  DNA
<213>  artificial

<220>
<223>  hHBD2 cDNA

<400>  2
atgcgtgttc tgtatctgct gttctctttc ctgttcatct tcctgatgcc tttgcctggt      60

gtattcggta tgggcattgg tgatccagtc acctgcctga aatccggtgc catctgtcat     120

cctgtgttct gtcctcgtcg ttacaagcaa attggtactt gcggtttgcc aggcaccaag     180

tgctgtaaga agccataatg a                                               201


<210>  3
<211>  198
<212>  DNA
<213>  artificial

<220>
<223>  nHBD2 cDNA

<400>  3
atgcgtgttc tgtatctgct gttctctttc ctgttcatct tcctgatgcc gctgccgggt      60

gtgtttggtg gcattggtga tccggtgacc tgcctgaaaa gcggtgccat ctgtcatccg     120

gtgttttgtc cgcgtcgtta taaacagatt ggtacctgcg gtctgccggg caccaaatgc     180

tgtaaaaaac cgtaatga                                                   198


<210>  4
<211>  21

<212>  DNA
<213>  artificial

<220>
<223>  Primer P1

<400>  4
ctttaagaat tcatgcgtgt t                                                21


<210>  5
<211>  22
<212>  DNA
<213>  artificial

<220>
<223>  Primer P2

<400>  5
cttattaagc tttcattacg gt                                               22


<210>  6
<211>  19
<212>  DNA
<213>  artificial

<220>
<223>  Primer P3

<400>  6
ctgccgggtg aattcggtg                                                   19


<210>  7
<211>  19
<212>  DNA
<213>  artificial

<220>
<223>  Primer P4

<400>  7
tcaggaggcc ggatccatg                                                   19


<210>  8
<211>  22
<212>  DNA
<213>  artificial

<220>
<223>  Primer P5

<400>  8
gcaggaattc gatatcaagc tt                                               22


<210>  9
<211>  18
<212>  DNA
<213>  artificial

<220>
<223>  Primer P6

<400>  9
gtaccagcgg atcccagg                                                    18

<210>  10
<211>  20
<212>  DNA
<213>  artificial

<220>
<223>  Primer P7

<400>  10
tagagagctc ccatggagaa                                                    20


<210>  11
<211>  23
<212>  DNA
<213>  artificial

<220>
<223>  Primer P8

<400>  11
gcgttaatga gatatcctcg aga                                                23


<210>  12
<211>  285
<212>  DNA
<213>  artificial

<220>
<223>  HD5-gene

<400>  12
atgaggacca tcgccatcct tgctgccatt ctcctggtgg ccctgcaggc ccaggctgag        60

tcactccagg aaagagctga tgaggctaca acccagaagc agtctgggga agacaaccag       120

gaccttgcta tctcctttgc aggaaatgga ctctctgctc ttagaacctc aggttctcag       180

gcaagagcca cctgctattg ccgaaccggc cgttgtgcta cccgtgagtc cctctccggg       240

gtgtgtgaaa tcagtggccg cctctacaga ctctgctgtc gctga                       285


<210>  13
<211>  288
<212>  DNA
<213>  artificial

<220>
<223>  nHD5-gene

<400>  13
atgcgtacca ttgccattct ggccgccatt ctgctggtgg ccctgcaggc ccaggccgaa        60

agcctgcagg aacgtgccga tgaagccacc acccagaaac agagcggtga agataatcag       120

gatctggcca ttagctttgc cggtaatggt ctgagcgccc tgcgtaccag cggtagccag       180

gcccgtgcca cctgctattg ccgtaccggt cgttgcgcca cccgtgaaag cctgagcggt       240

gtgtgcgaaa ttagcggtcg tctgtatcgt ctgtgctgcc gttaatga                    288

## Description

### FIELD OF THE INVENTION

The present invention is directed to a recombinant *Escherichia coli (E. coli)* strain Nissle 1917 (EcN) cell transformed with a nucleic acid coding for a defensin protein or a derivative thereof. The invention is further directed to a pharmaceutical composition comprising this cell and a pharmaceutically acceptable carrier as well as a method of producing a recombinant *E. coli* Nissle 1917 cell and its use in the treatment of Crohn's disease.

### BACKGROUND OF THE INVENTION

*Escherichia coli* strain Nissle 1917 (EcN) is one of the best studied probiotic strains and the active component of the microbial drug Mutaflor^{®} (Ardeypharm GmbH, Herdecke, Germany). This probiotic drug is successfully used in several European countries for the treatment of various diseases of the digestive tract, including acute and prolonged diarrhea, uncomplicated diverticular disease, and inflammatory bowel disease (IBD). It is particularly used for maintenance therapy of remission in patients with ulcerative colitis (UC). In ulcerative colitis, it is thought that one of the main mechanisms of action of EcN is the induction of defensin synthesis in intestinal epithelial cells. The molecular mechanism behind the clinical effectiveness of EcN in UC-treatment might be the flagellin-mediated enhancement of human β-defensin 2 (HBD2) production which is due to induction of a NF-kB- and AP-1-dependent signaling pathway.

On the other hand, except in small-sized clinical trials, EcN has not been shown to be generally effective in Crohn's disease (CD) patients. The reason is that the low amount of antimicrobial peptides, especially defensins, in the gut of patients with CD appears to be due to a primary defect of defensin synthesis. It was reported that mutations in the NOD2 (nucleotide-binding oligomerization domain 2) gene, an intracellular receptor for bacterial muramyl dipeptide, are clearly associated with ileal Crohn's disease. Wehkamp and colleagues also found a pronounced decrease of α-defensin transcripts in patients with Crohn's disease carrying a NOD2 mutation as compared to the wild type (Wehkamp et al., 2004b, Wehkamp et al., 2005). Moreover, Wehkamp's research group was able to show a reduced expression of TCF4 which is a known regulator of Paneth cell differentiation and also α-defensin expression, in patients with ileal Crohn's disease as compared to colonic Crohn's disease and ulcerative colitis (van Es et al. 2005; Wehkamp et al., 2007). Besides, the number of HBD2 gene copies was shifted to lower numbers in colonic CD as compared to controls (3 vs. 4) (Fellermann et al., 2006).

Defensins are cationic, arginine-rich, small peptides between 3.5 and 4 kDa in size with six cysteines that form three disulfide bridges. They exhibit a broad-spectrum antimicrobial activity against bacteria, fungi and some enveloped viruses and can also act as chemokines. Their mechanism of antimicrobial action is an attachment of the cationic defensins to the negatively charged bacterial cell surface resulting in a membranolytic disruption of the plasma membrane. Defensins are classified as α- or ß-defensins based on the position of the 3 intramolecular disulfide bridges. 6 α-defensins (HNP 1-4, HD5 and HD6) and 6 ß-defensins (HBD1 to 6) have been identified in humans. Human α-defensins provide antimicrobial host defense throughout the periphery and small intestine by their expression and release from granulocytic neutrophil and Paneth cell populations. Human α-defensins are synthesized as larger, inactive pro-peptide molecules and the mature, active peptides result from post-translational processes. Human α-defensin 5 (HD5) is primarily expressed in intestinal Paneth cells as an inactive pro-peptide. It is cleaved into the active mature HD5 by trypsin stored in Paneth cell granules. Human ß-defensins are produced by epithelial cells that are part of both the innate and adaptive immune responses. In contrast to human α-defensins, human ß-defensins are not expressed as pro-forms. HBD2 synthesis is induced in the skin as well as in urinary, gastrointestinal, and respiratory epithelia through stimulation of epithelial cells by contact with microorganisms or cytokines, such as TNF-α and IL-1β.

Small cationic antimicrobial peptides have already been successfully synthesized in *E. coli* by recombinant DNA methods. However, there are several pitfalls using *E. coli* as the host cell for cationic antimicrobial peptide expression, such as the host-killing activity of the product and its susceptibility to proteolytic degradation. The fusion expression of the target peptide with a partner alleviates these problems. However, there arises another problem in that most fusion products are inactive or produced in an insoluble form. It has been reported that a fusion expression system with thioredoxin (TrxA) resulted in an increased yield of soluble products. This has also been observed for HBD2 and HBD3. As mentioned above, the gut of CD patients possesses a low amount of antimicrobial peptides, especially defensins, because of a primary defect of defensin synthesis.

Therefore, it is an object of the present invention to provide a new therapeutic system which allows a convenient in vivo application of physiologically acceptable cells expressing defensins. It is a further object of the invention to provide a therapeutic approach which overcomes the physiological deficiencies in endogenous defensin synthesis in patients in order to provide an effective treatment of patients suffering from Crohn's Disease. It is a further object of the present invention to provide recombinant *E. coli* cells comprising nucleic acids coding for a defensin protein and which are capable of delivering defensin proteins to an intended target site in the human body. It is a further object of the present invention to provide a host cell which is able to resist the host-killing activity of defensin proteins.

### SUMMARY OF THE INVENTION

These objects are achieved by the recombinant *E. coli* Nissle 1917 (EcN) cells according to the invention which have been transformed with a nucleic acid coding for a defensin protein or a derivative thereof. The present inventors successfully constructed defensin-producing and -secreting probiotic EcN bacteria for supplementing the lacking endogenous defensin synthesis in human patients.

The inventors unexpectedly found out that *E. coli* strain Nissle 1917 may express defensin recombinantly in a therapeutically suitable amount without being affected by the host-killing activity of the defensin protein.

Therefore, a new pharmaceutical composition is provided comprising recombinant *E. coli* Nissle 1917 cells encoding the defensin proteins or a derivative thereof as well as the use thereof in the treatment of Crohn's disease.

The present invention is further directed to a method of producing such a recombinant *E. coli* Nissle 1917 cell which method comprises the steps of providing a nucleic acid coding for a defensin protein or a derivative thereof and *E. coli* Nissle 1917 cells; and transforming the cells with said nucleic acid, where the transformed *E. coli* Nissle 1917 cells are capable of expressing a defensin protein or derivative thereof.

### DETAILED DESCRIPTION OF THE INVENTION

According to a first aspect, the present invention is directed to a recombinant *E. coli* Nissle 1917 cell transformed with a nucleic acid coding for a defensin protein or a derivative thereof.

The term "nucleic acid sequence" in the context of the present invention means a heteropolymer of nucleotides or the sequence of these nucleotides. The term "nucleic acid" comprises RNA as well as DNA, including cDNA, genomic DNA and synthetic (e.g. chemically synthesized) nucleic acids and the like.

The term "derivative", as used herein, means a derivative of a nucleic acid coding for a defensin protein which contains one or more substitutions, insertions and/or deletions when compared to the original defensin nucleic acid sequence. Those derivatives preferably lack one, but also 2, 3, 4 or more nucleotides 5'- or 3'- or within the nucleic acid sequence, or the nucleotides are replaced by others.

The term derivative in particular comprises those nucleic acids which are in essence equivalent to the original nucleic acids encoding defensin but showing at least about 80%, more typically at least about 90% or 95% sequence identity to the original defensin encoding sequences.

In particular variants of the protein, for example deletions, insertions and/or substitutions in the sequence, which cause for so-called "silent" changes, are considered to be part of the invention. For example, such changes in the nucleic acid sequence are considered to cause a substitution with an equivalent amino acid. Preferably are such amino acid substitutions the result of substitutions which substitute one amino acid with a similar amino acid with similar structural and/or chemical properties, i.e. conservative amino acid substitutions.

Amino acid substitutions can be performed on the basis of similarity in polarity, charges, solubility, hydrophobic, hydrophilic, and/or amphiphilic nature of the involved residues. Examples for hydrophobic amino acids are alanine, leucine, isoleucine, valine, proline, phenylalanine, tryptophan and methionine. Polar, neutral amino acids include glycine, serine, threonine, cysteine, thyrosine, asparagine and glutamine. Positively (basic) charged amino acids include arginine, lysine and histidine. And negatively charged amino acids include aspartic acid and glutamic acid.

"Insertions" or "deletions" usually range from one to five amino acids. The allowed degree of variation can be experimentally determined via methodically applied insertions, deletions or substitutions of amino acids in a protein molecule using recombinant DNA methods. The resulting variants can be tested for their biological activity.

Nucleotide changes, which affect the N-terminal and C-terminal part of the protein, often do not change the protein activity, because these parts are often not involved in the biological activity. It can be desired to eliminate one or more of the cysteines of the sequence, since cysteines can cause the unwanted formation of multimers when the protein is produced recombinantly. Multimers may complicate purification procedures. Each of the suggested modifications is in range of the current state of the art, and under the retention of the biological activity of the encoded products.

"Biological activity" in the context of the defensin proteins in particular means their antimicrobial activity against bacteria, fungi and some enveloped viruses. It is further noted that whenever the present invention mentions a "derivative" of a defensin protein, a protein is meant which is having a biological function equivalent to the original defensin protein, i.e. the anti-microbial action and its usefulness in the treatment of Crohn's Disease, i.e. for supplementing the lacking endogenous defensin synthesis.

In a preferred embodiment, the recombinant *E. coli* cell of the present invention is transformed with a nucleic acid coding for a defensin protein selected from human α- or β-defensin. Those defensin proteins more preferably are selected from human α-defensins HNP1-4, human α-defensin 5 (HD5), human α-defensin 6 (HD6) and human β-defensins 1-6 (HBD1-6) or from the respective mature form of said proteins.

The term "mature form of said proteins" refers to human α-defensins only which are expressed in pro-forms which, following cleavage, result in the active mature form of α-defensin. Human β-defensins are not expressed as pro-forms.

The sequences of human α- and β-defensins as outlined above have been published before and are disclosed, for example, in the following databases:

### Human defensin sequences

### Human α-defensins

Human α-defensin 1
   http://www.ncbi.nlm.nih.gov/nuccore/NM 004084.3
Human α-defensin 3
   http://www.ncbi.nlm.nih.gov/nuccore/NM 005217.3
Human α-defensin 4
   http://www.ncbi.nlm.nih.gov/nuccore/NM 001925.1
Human α-defensin 5
   http://www.ncbi.nlm.nih.gov/nuccore/NM 021010.1
Human α-defensin 6
   http://www.ncbi.nlm.nih.gov/nuccore/NM_001926.3

### Human β-defensins

Human β-defensin
   http://www.ncbi.nim.nih.gov/nuccore/NM 005218.3

Human β-defensin 2
   http://www.ncbi.ntm.nih.gov/nuccore/af040153
Human β-defensin 3
   http://www.ncbi.nlm.nih.gov/nuccore/AF301470.1
Human β-defensin 4
   http://www.ncbi.nlm.nih.gov/nuccore/BC093983.1
Human β-defensin 5
   http://www.ncbi.nlm.nih.gov/nuccore/AB089180.1
Human β-defensin 6
   http://www.ncbi.nlm.nih.gov/nuccore/AB089181.1

In a further preferred embodiment, the defensin protein or derivative thereof is, according to the present invention, expressed as a fusion protein, preferably as a fusion protein with a HIS-tag or with an *E. coli* YEBF protein. *E. coli* protein YEBF (10.8 kDa) is a soluble endogenous protein secreted into the extracellular medium and has been used as a carrier for transgenic proteins in the past. Passenger proteins, such as defensin, linked to the carboxyl end of YEBF are efficiently secreted by the respective recombinant *E. coli* strain. That is to say, a fusion protein of defensin and YEBF can be efficiently secreted from *E. coli* cells leading to a higher amount of secreted defensin than from *E. coli* cells which are expressing the defensin protein in pure form.

In a further embodiment, the nucleic acid coding for a defensin protein or the derivative thereof is adapted for expression in *E. coli* cells by codon optimization. Codon optimization is a technique used to improve the protein expression in living organisms by increasing the translational efficiency of the gene of interest. In particular, it is known that codon optimization can improve the expression of human genes (such as defensin) in *E. coli* cells. Thus, the efficiency of expression is optimized by using the most common host codons for gene expression. Preferred examples of nucleic acids coding for defensin proteins being adapted for expression in *E. coli* Nissle 1917 cells are those according to sequence ID NOs: 1, 2 and 3. SEQ ID NO: 1 is the original HBD2 cDNA, which has not been optimized, whereas SEQ ID NOs: 2 and 3 are optimized as hHBD2 and nHBD2 sequences. SEQ ID NOs: 2 and 3 are most preferred for expression in *E. coli* Nissle 1917 cells.

In a further embodiment, the nucleic acid coding for the defensin protein or derivative thereof is part of an expression vector, which expression vector additionally contains one or more regulatory sequences. Numerous vectors are known to be appropriate for the transformation of bacterial cells: for example, plasmids and bacteriophages, like the phage λ, are frequently used as vectors for bacterial hosts.

In a preferred embodiment, the expression vector is a plasmid and the regulatory sequences comprise the T7 RNA polymerase promoter.

In a further aspect, the present invention is directed to a pharmaceutical composition comprising recombinant *E. coli* Nissle 1917 cells as defined above and a pharmaceutically acceptable carrier.

The active components of the present invention thus are preferably used in such a pharmaceutical composition in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutical acceptable" defines a non-toxic material, which does not interfere with effectiveness of the biological activity of the active component. The choice of the carrier is dependent on the application.

The pharmaceutical composition can contain additional components which enhance the activity of the active component or which supplement the treatment. Such additional components and/or factors can be part of the pharmaceutical composition to achieve a synergistic effect or to minimize adverse or unwanted effects.

General techniques for the formulation or preparation and application/medication of compounds of the present invention may be found in "Remington's Pharmaceutical Sciences", Mack Publishing Co., Easton, PA, latest edition.

For example, the transformed *E. coli* cells of the present invention may be administered orally, for example encapsuled in a hard gelatine capsule. Such a capsule may contains about 2,5 - 25 x 10⁹ viable *E. coli* cells as a single dosage. Further ingredients might be selected from usual auxiliaries such as maltodextrine, talc, Eudragit® L 100, macrogol (4000), triethylcitrat, glycerol (just to name a few).

In a further aspect, the present invention is directed to a recombinant *E. coli* Nissle 1917 (EcN) cell as defined above for use in the treatment of Crohn's disease.

An additional aspect of the invention is a method of producing a recombinant *E. coli* Nissle 1917 cell comprising the steps of providing a nucleic acid coding for a defensin protein or derivative thereof and *E. coli* Nissle 1917 cells, and transforming the cells with said nucleic acid, where the transformed *E. coli* Nissle 1917 cells are capable of expressing a defensin protein or derivative thereof.

The nucleic acid preferably is cloned into an expression vector and introduced into the cell by electroporation. As outlined above, the defensin protein or derivative thereof is expressed as a fusion protein with an N-terminal His-tag and/or as a fusion protein with the *E. coli* YebF protein.

Unless otherwise defined, all technical and scientific terms used herein have the same meaning as commonly understood by one of ordinary skill in the art to which this invention pertains. All publications mentioned herein are incorporated by reference in their entirety. In case of conflict, the present specification, including definitions, will control. In addition, the materials, methods, and examples are illustrative only and not intended to be limiting.

The invention is now further illustrated by the accompanying figures.

### BRIEF DESCRIPTION OF THE FIGURES

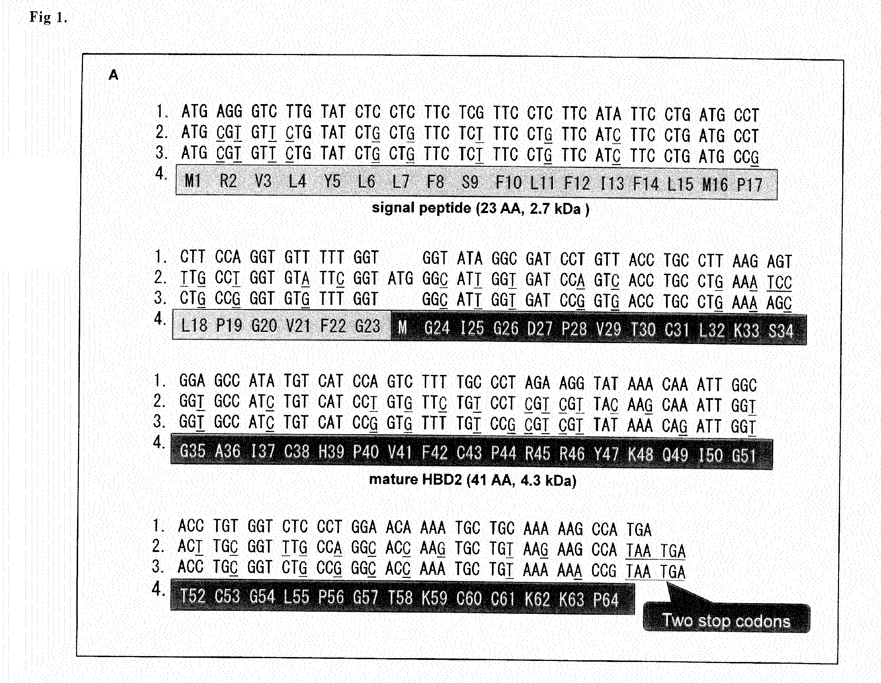

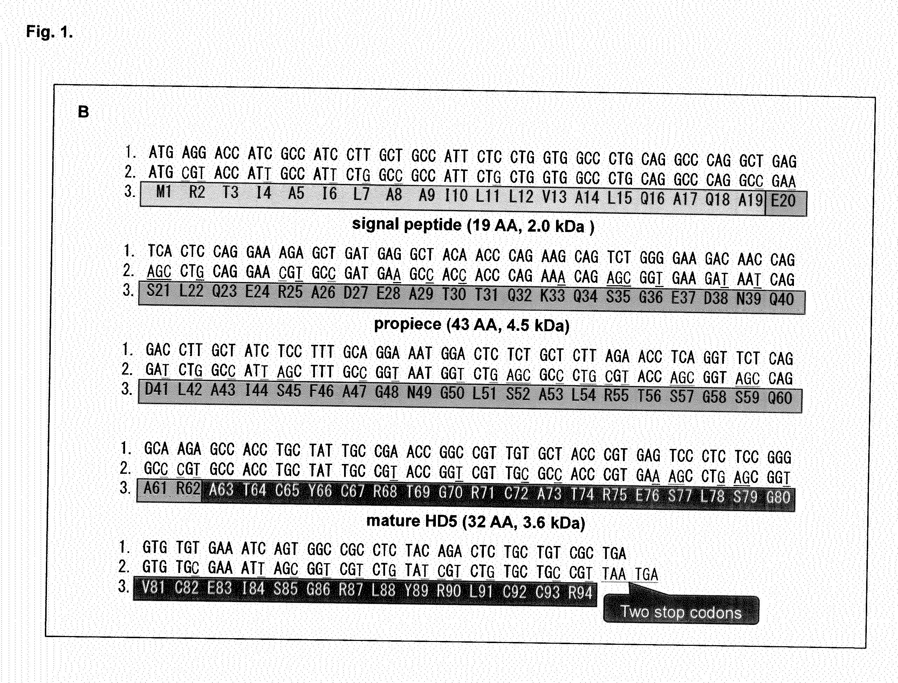

Fig. 1. Comparison of (A) HBD2 and (B) HD5 sequences between original cDNA and modified ones, lane 1: original sequences of the cDNA, (A) HBD2-gene (GeneBank accession No AF040153) and (B) HD5-gene (GeneBank accession No M97925) sequences; lane 2 of (A): optimized sequences of shHBD2; lane 3 of (A): optimized sequences of nHBD2-gene (EMBL HE583189); lane 4 of (A): sequences of corresponding amino acids of HBD2; and lane 2 of (B): nHD5-gene (EMBL HE583188); lane 3 of (B): sequences of corresponding amino acids of and (B) HD5.

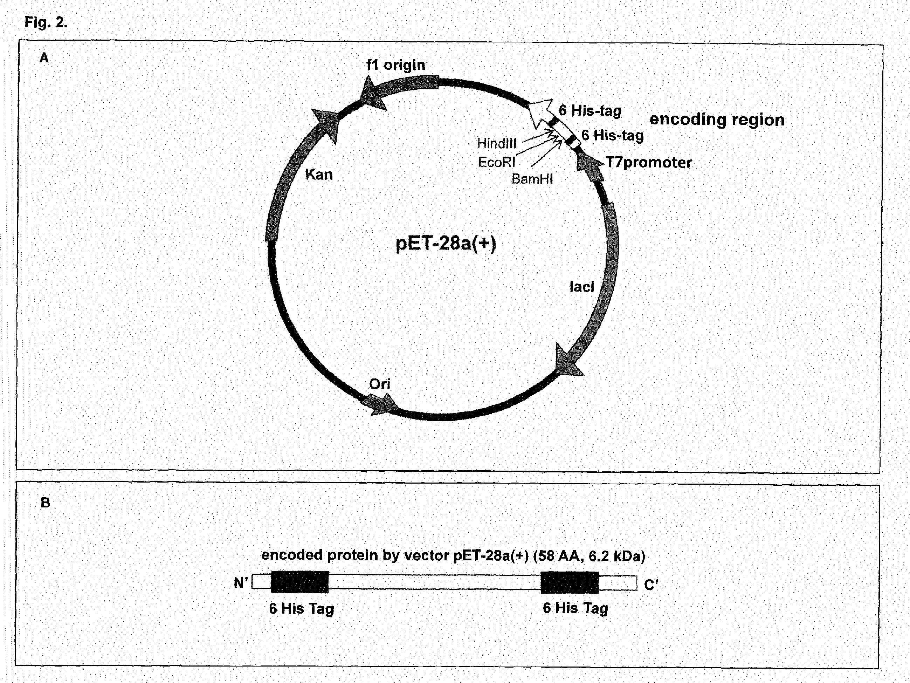

Fig. 2. Expression vector pET-28a(+). (A) Physical map of vector pET-28a(+) harboring the gene (open arrow in A) encoding the protein depicted in. (B) Schematic composition of the protein encoded by vector pET-28a(+).

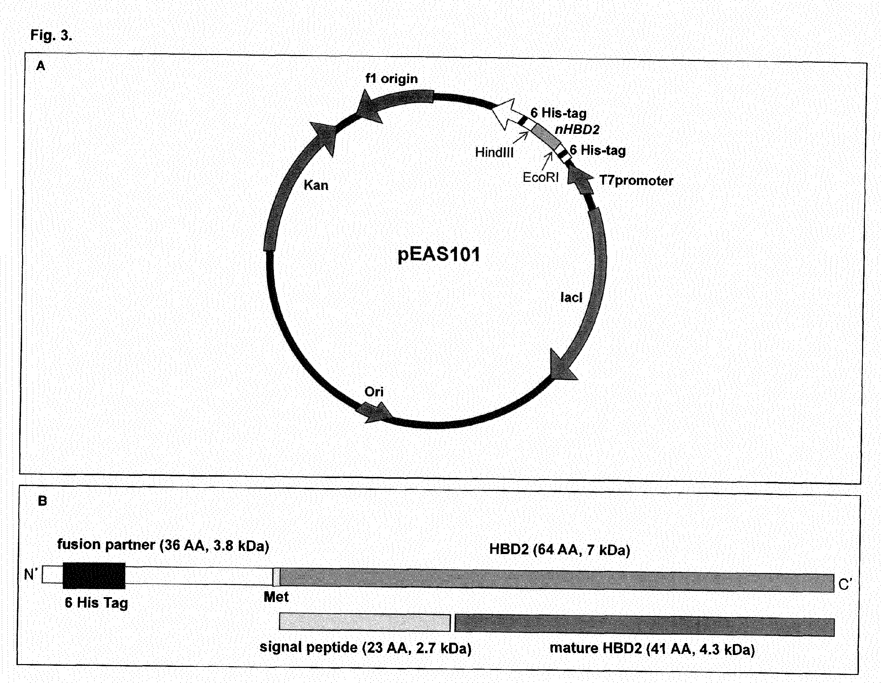

Fig. 3. Expression plasmid pEAS101. (A) Physical map of the recombinant plasmid pEAS101 encoding the fusion gene composed of the fusion partner including a His-tag and the nHBD2-gene. (B) Schematic composition of the fusion protein HisHBD2.

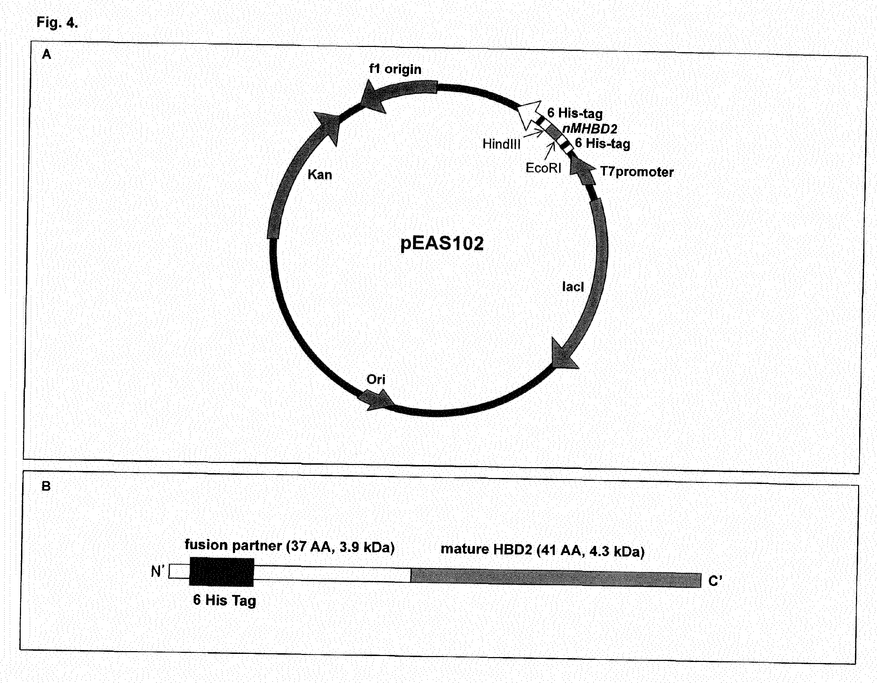

Fig. 4. Expression plasmid pEAS102. (A) Physical map of the recombinant plasmid pEAS102 harboring the fusion gene composed of the fusion partner including a His-tag and that part of the nHBD2-gene encoding the mature form of HBD2. (B) Schematic composition of the fusion protein HisMHBD2.

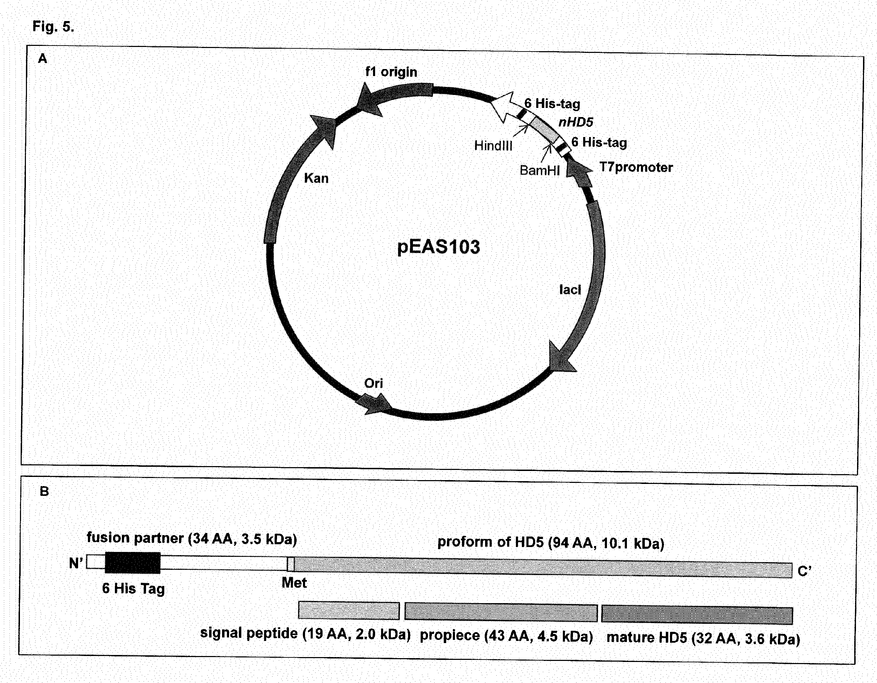

Fig. 5. Expression plasmid pEAS103. (A) Physical map of pEAS103 containing the fusion gene composed of the fusion partner with a His-tag and the nHD5-gene. (B) Physical map of the composition of the fusion protein HisHD5.

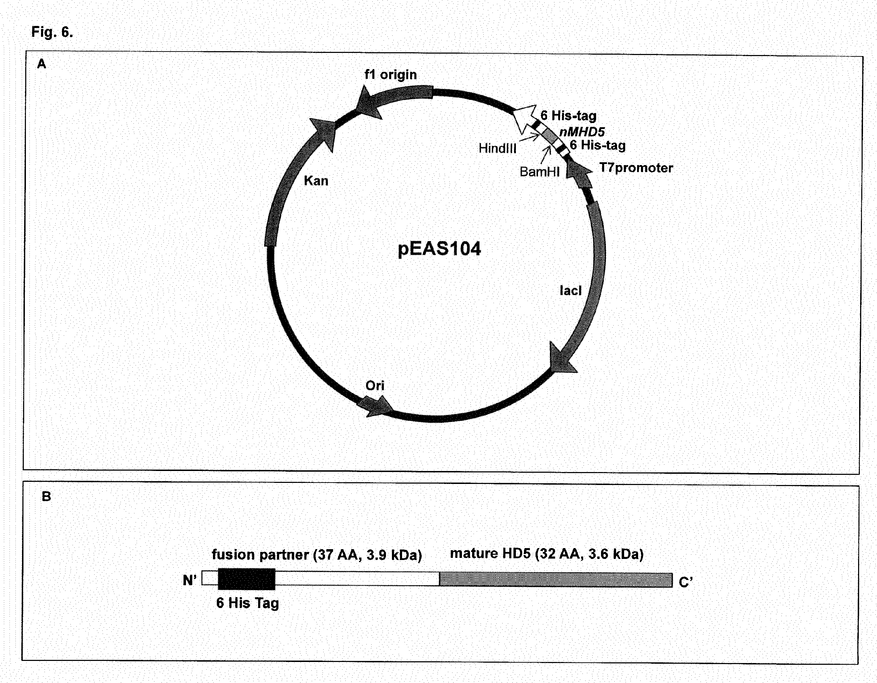

Fig. 6. Expression plasmid pEAS104. (A) Physical map of the recombinant plasmid pEAS104 encoding the fusion gene composed of the fusion partner encoding a His-tag and that part of the nHD5-gene encoding mature HD5. (B) Fusion protein HisMHD5 and the parts it is composed of.

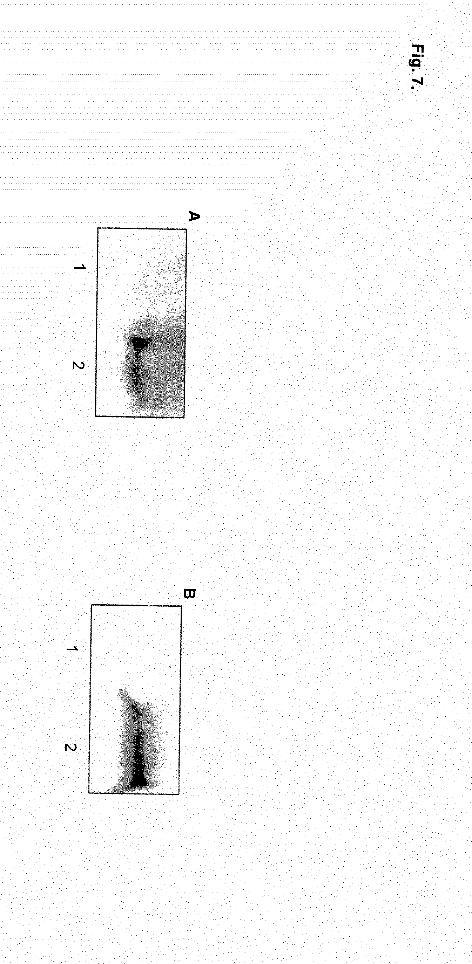

Fig. 7.(A) Western blot analysis with anti-HBD2 rabbit polyclonal antiserum of whole cell proteins after induction of expression at 20 °C for 6 h (Samples were pellet of whole cell proteins after centrifugation and adjusted to cell density (1.5x10⁸ cfu cells were loaded for each lane)). Lane 1: EcN100, Lane 2: EcN101. (B) Western blot analysis with anti-HBD2 rabbit polyclonal antiserum of soluble and insoluble forms of HisHBD2 expressed in EcN101 after induction at 20 °C for 6 h (Samples were adjusted to the number of bacterial cells (1.5x10⁸ cfu)). Lane 1: supernatant of whole cell proteins after cell lysis and centrifugation and filtration (i.e. soluble protein fraction); Lane 2: pellet of whole cell proteins after cell lysis and centrifugation (i.e. insoluble protein fraction).

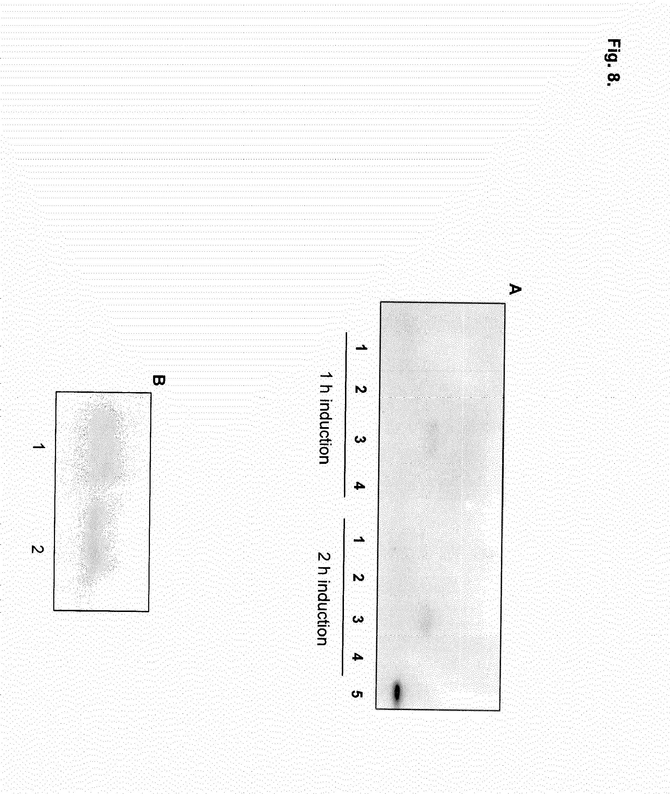

Fig. 8.(A) Western blot analysis with anti-HBD2 rabbit polyclonal antiserum of whole cell proteins after induction of expression at 37 °C (Samples were adjusted to the number of bacterial cells (1.5x10⁸ cfu)). Lane 1: EcN100, pellet of whole cell proteins after centrifugation; Lane 2: EcN100, cell-free culture medium after centrifugation and filtration; Lane 3: EcN102, pellet of whole cell proteins after centrifugation; Lane 4: EcN102, cell free culture medium after centrifugation and filtration; Lane 5: positive control (commercial HBD2, 1 µg). (B) Western blot analysis with anti-HBD2 rabbit polyclonal antiserum of soluble and insoluble forms of HisMHBD2 expressed in EcN102 after induction at 37 °C for 2 h (Samples were adjusted to the number of bacterial cells (1.5x10⁸ cfu)). Lane 1: supernatant of whole cell proteins after cell lysis and centrifugation and filtration; Lane 2: pellet of whole cell proteins after cell lysis and centrifugation.

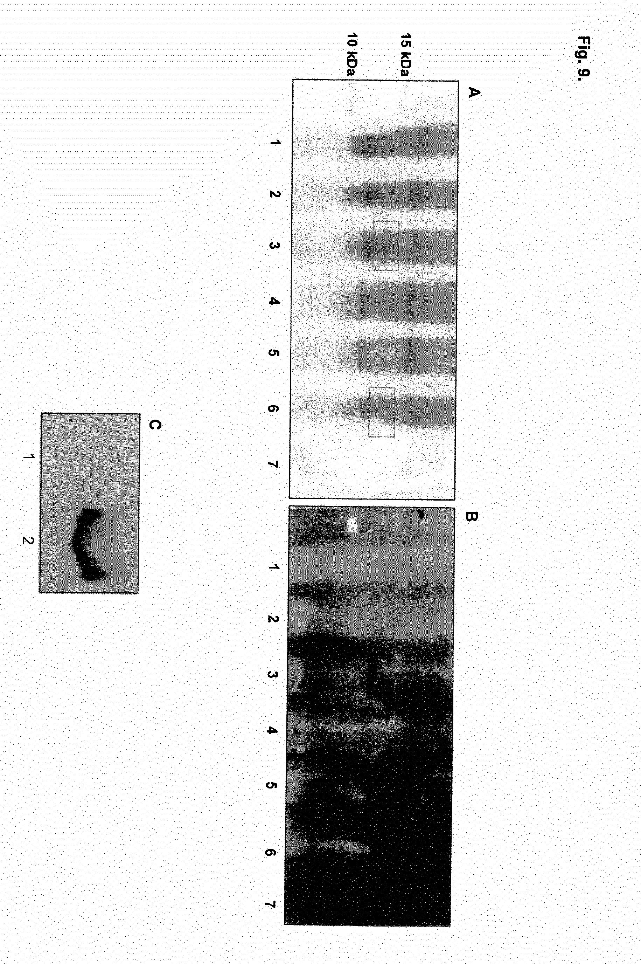

Fig. 9.(A) Silver-stained gel after SDS-PAGE (the boxes indicate expressed HisHD5) and (B) Western blot analysis with anti-HD5 rabbit polyclonal antiserum of whole cell proteins after induction of expression at 37 °C for 4 h (Samples were pellet of whole cell proteins after centrifugation, and identical optical densities (corresponding to 1.5x10⁸ cfu) were loaded for each lane). Lane 1: EcN1219, Lane 2: EcN100, Lane 3: EcN103, Lane 4: EcN1219, Lane 5: EcNc100, Lane 6: EcNc103, Lane 7: positive control (commercial HD5, 1 µg). (C) Western blot analysis with anti-HD5 rabbit polyclonal antiserum of soluble and insoluble forms of HisHD5 expressed in EcN103 after induction at 37 °C for 4 h (Samples were adjusted to the number of bacterial cells (1.5x10⁸ cfu)). Lane 1: supernatant of whole cell proteins after cell lysis and centrifugation and filtration (i.e. the soluble protein fraction); Lane 2: pellet of whole cell proteins after cell lysis and centrifugation (i.e. the insoluble protein fraction).

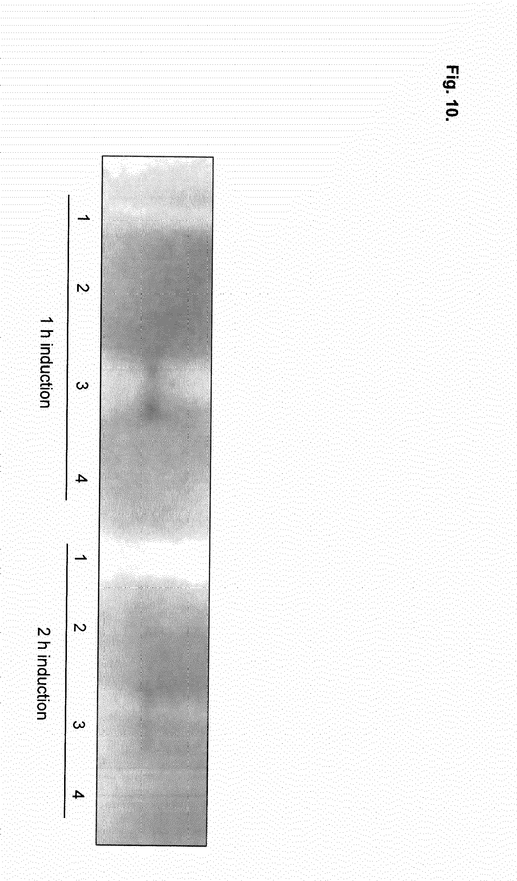

Fig. 10. Western blot analysis with anti-HD5 rabbit polyclonal antiserum of whole cell proteins after induction at 37 °C (Samples were adjusted to the number of bacterial cells (1.5x10⁸ cfu)). Lane 1: EcN100, pellet of whole cell proteins after centrifugation; Lane 2: EcN100, cell-free culture medium after centrifugation and filtration; Lane 3: EcN104, pellet of whole cell proteins after centrifugation; Lane 4: EcN104, cell-free culture medium after centrifugation and filtration.

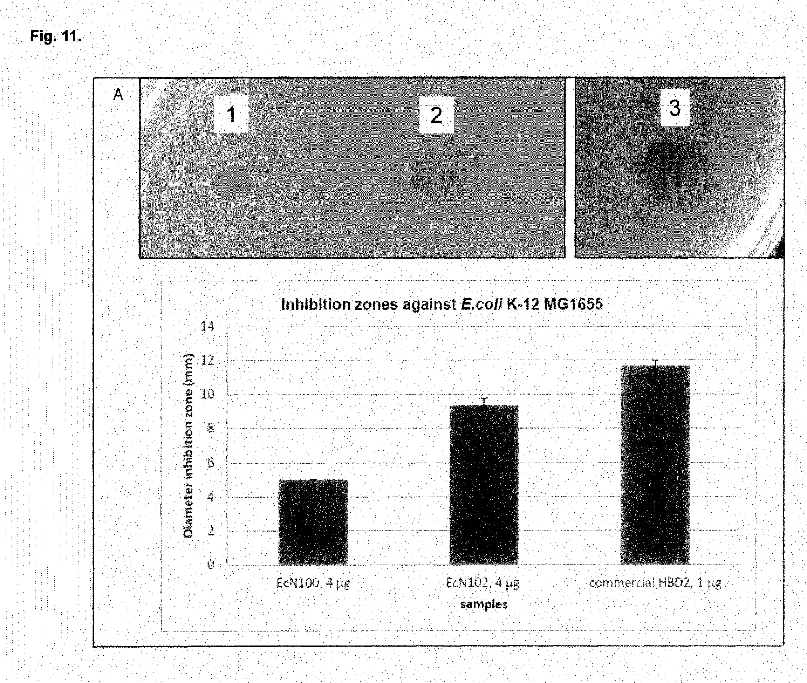

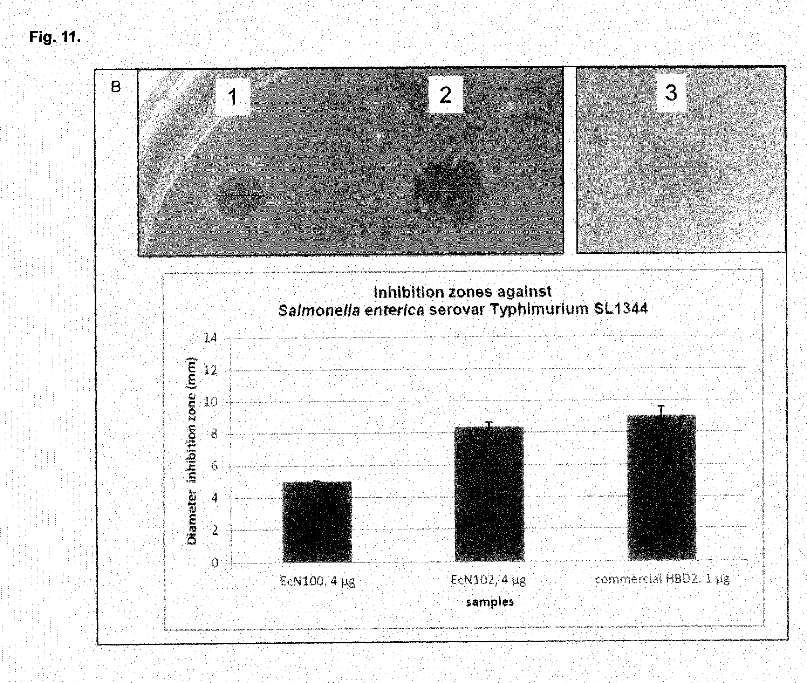

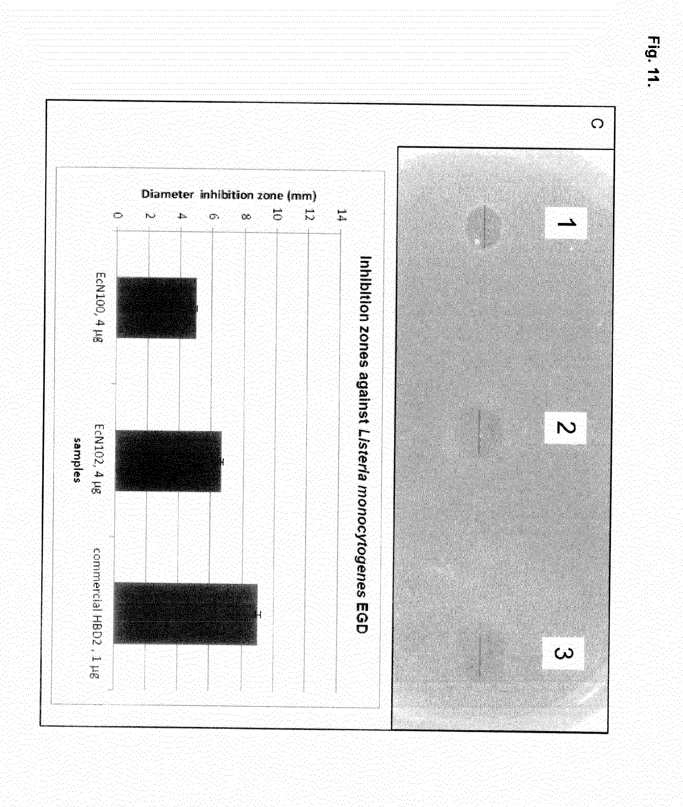

Fig. 11. The antimicrobial activity of HisMHBD2 against (A) *E. coli* K-12 MG1655 and (B) *Salmonella enterica* serovar Typhimurium SL1344 and (C) *Listeria monocytogenes* EGD. 1: negative control (EcN100), 4 µg of fraction 0.03 eluted from Ni-column; 2: eluted proteins including HisHBD2 (EcN102), 4 µg of fraction 0.03 eluted from Ni-column; 3: positive control, commercial HBD2, 1 µg; The lines indicate the 5 mm diameters of wells, Triple experiments were carried out and the mean values and standard deviations are presented.

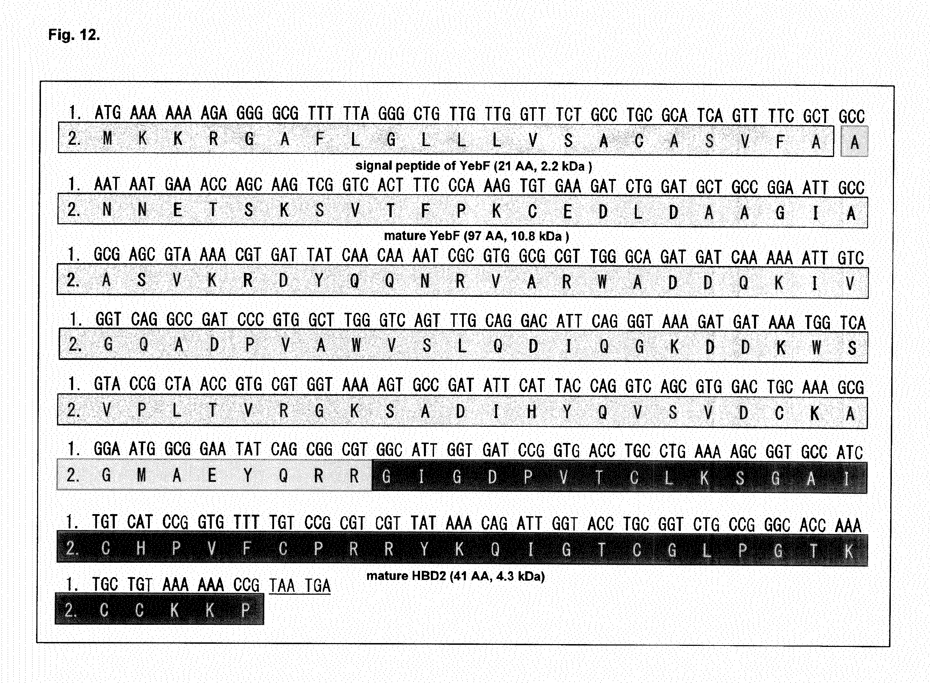

Fig. 12. DNA and amino acid sequences of the constructed fusion gene and the corresponding fusion protein YebFMHBD2; Lane 1: cDNA sequence of the yebFMHBD2-gene; Lane 2: sequence of the corresponding amino acids of YebFMHBD2.

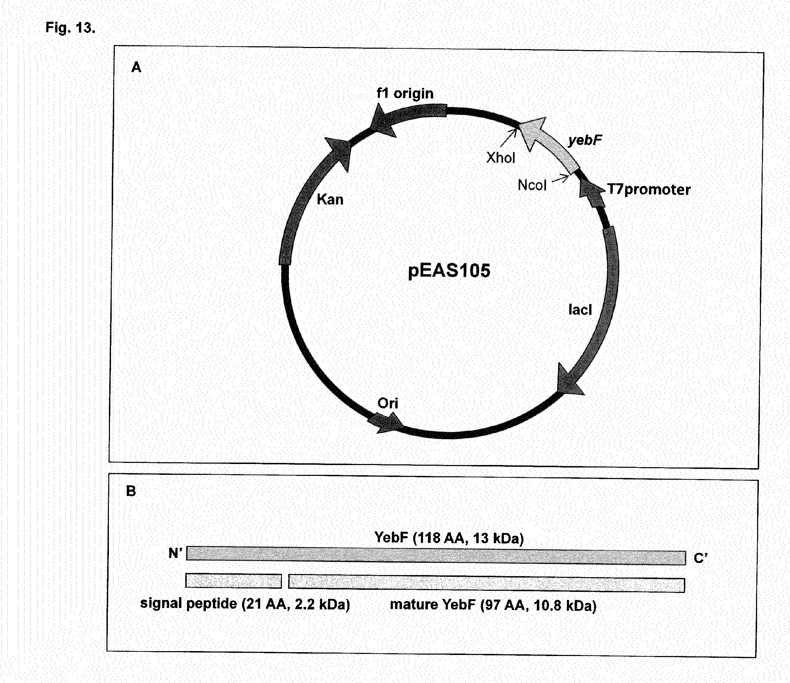

Fig. 13. Expression plasmid pEAS105. (A) Physical map of plasmid pEAS105 harboring the yebF gene. (B) Schematic presentation of the corresponding protein YebF.

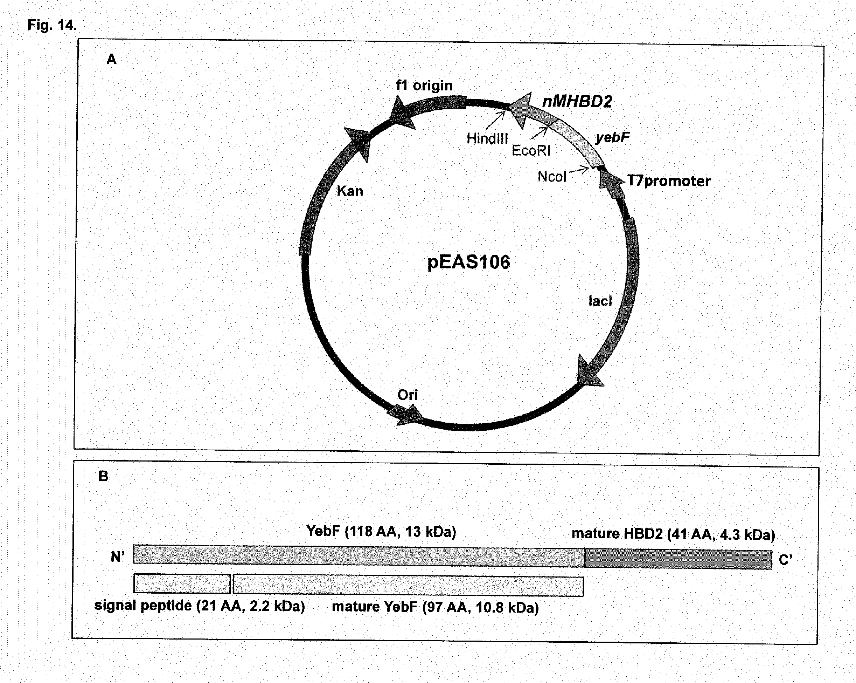

Fig. 14. Expression plasmid pEAS106. (A) Physical map of the recombinant plasmid pEAS106 encoding the fusion gene composed of the fusion partner, the yebF and the nMHBD2-gene. (B) Schematic composition of the fusion protein YebFMHBD2.

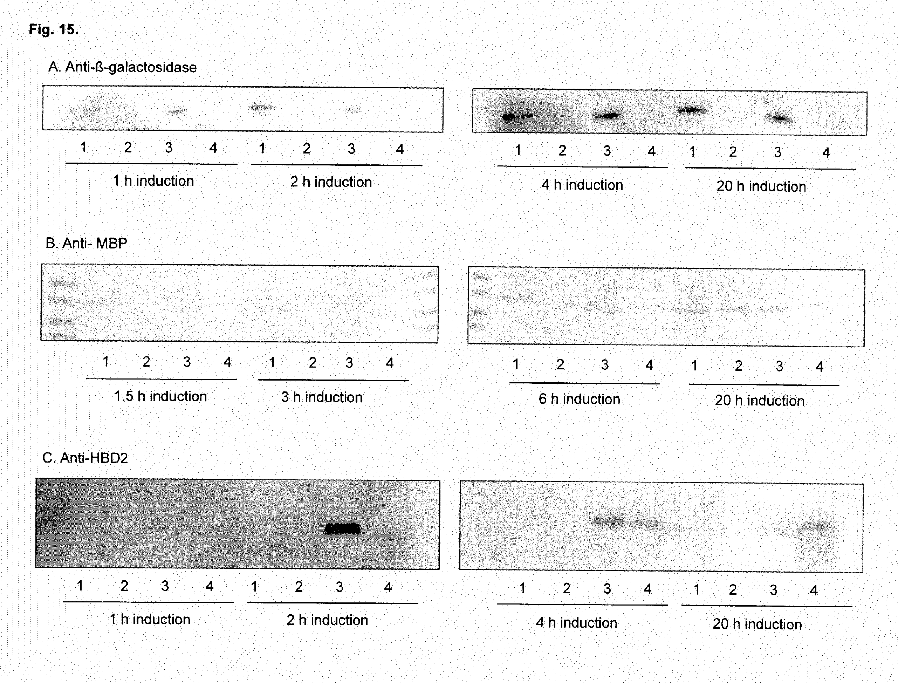

Fig. 15. Analysis of intracellular and secreted polypeptides from recombinant strains EcN105 and EcN106 (Samples were adjusted to the number of bacterial cells (1.1x10⁸ cfu)). Lane 1: EcN105, cellular proteins (pellet after centrifugation); Lane 2: EcN105, proteins in the cell-free supernatant (supernatant after centrifugation and filtration); Lane 3: EcN106, cellular proteins (pellet after centrifugation); Lane 4: EcN 106, proteins in the cell-free supernatant (supernatant after centrifugation and filtration).

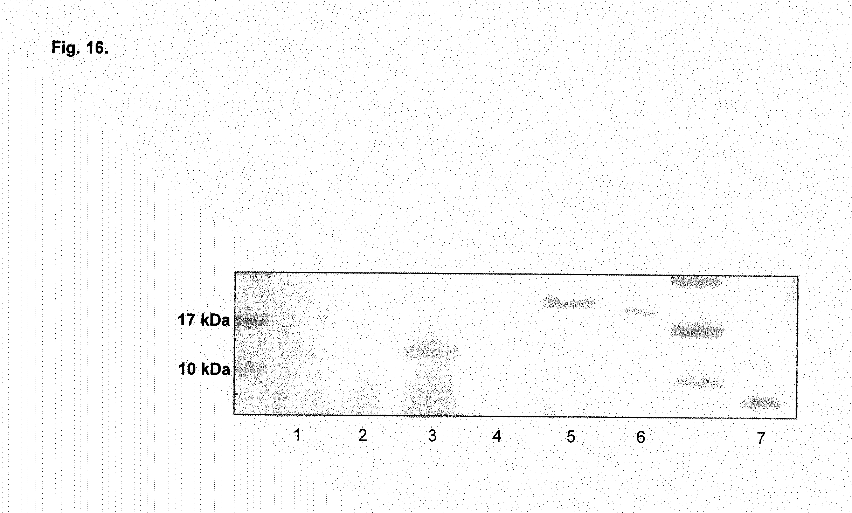

Fig. 16. Comparison of intracellular and secreted polypeptides from recombinant strains of EcN102 encoding HisMHBD2 and EcN106 encoding YebFMHBD2 at 2 h after induction by Western blot (Samples were adjusted to the number of bacterial cells (1.1x10⁸ cfu)). Lane 1: EcN100, cellular proteins (pellet after centrifugation); Lane 2: EcN100, proteins in the cell-free supernatant (supernatant after centrifugation and filtration); Lane 3: EcN102, cellular proteins (pellet after centrifugation); Lane 4: EcN102, proteins in the cell-free supernatant (supernatant after centrifugation and filtration); Lane 5: EcN106, cellular proteins (pellet after centrifugation); Lane 6: EcN106, proteins in the cell-free supernatant (supernatant after centrifugation and filtration); Lane 7: positive control, commercial HBD2, 1 µg, The theoretical molecular masses are: for HisMHBD2 8.2 kDa, for YebFMHBD2 17.3 kDa, for secreted YebFMHBD2 15.1 kDa, and for mature HBD2 4.3 kDa.

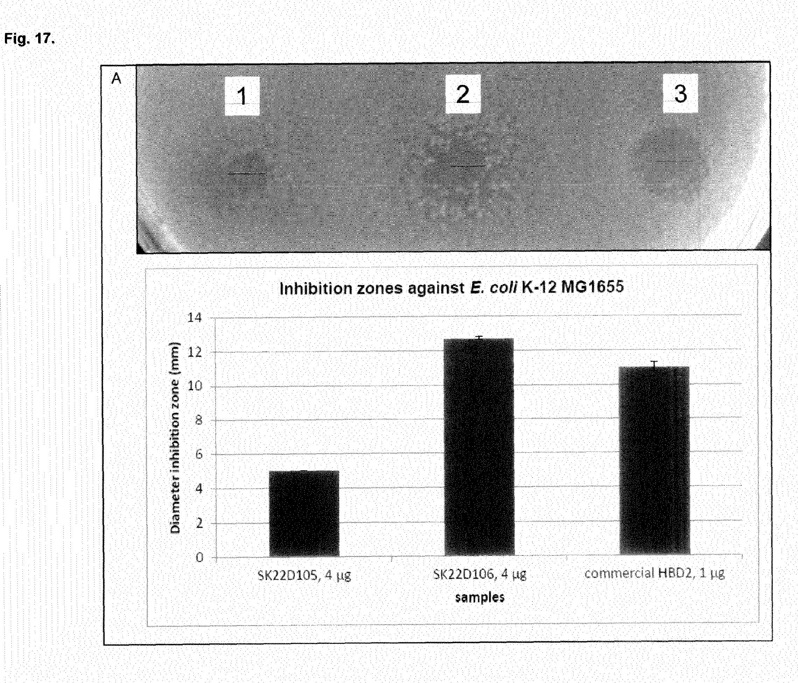

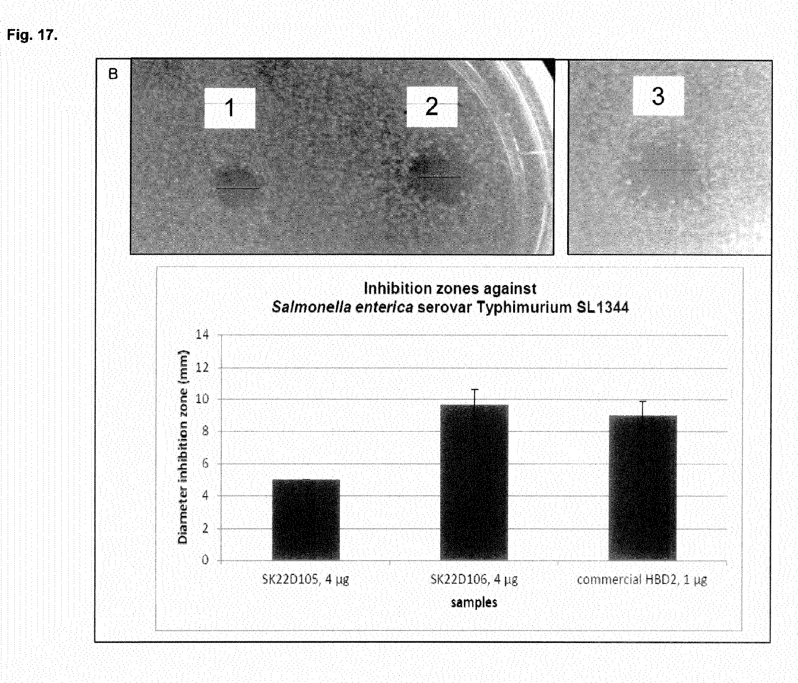

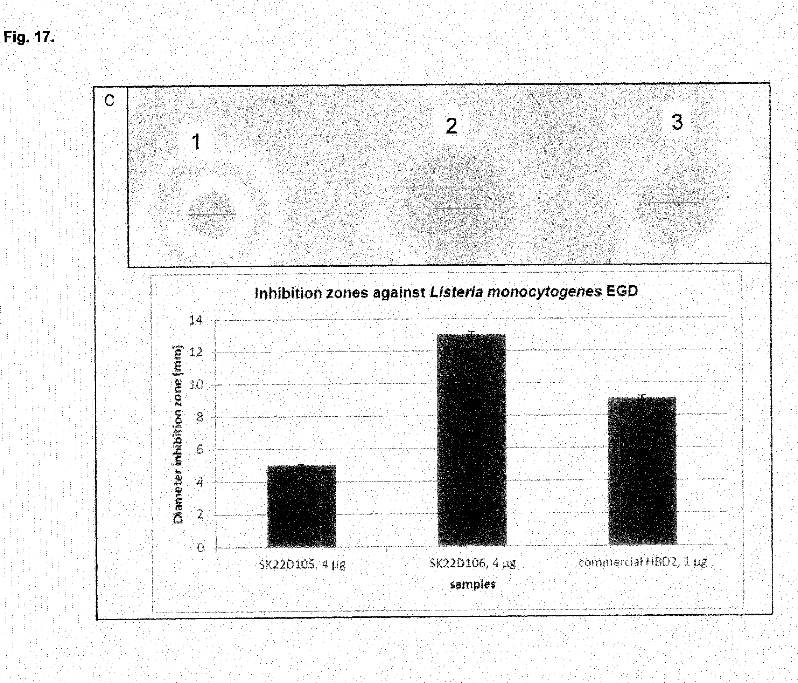

Fig. 17. The antimicrobial activity of YebFMHBD2 by radial diffusion tests with (A) *E. coli* K-12 MG1655 and (B) *Salmonella enterica* serovar Typhimurium SL1344 and (C) *Listeria monocytogenes* EGD; 1: negative control, proteins in the cell-free supernatant after centrifugation and filtration from SK22D105, 4 µg; 2: proteins in the cell-free supernatant including YebFMHBD2 after centrifugation and filtration from SK22D106, 4 µg; 3: positive control, commercial HBD2, 1 µg; The lines indicate the 5mm diameters of wells, Triple experiments were carried out and the mean values and standard deviations are presented.

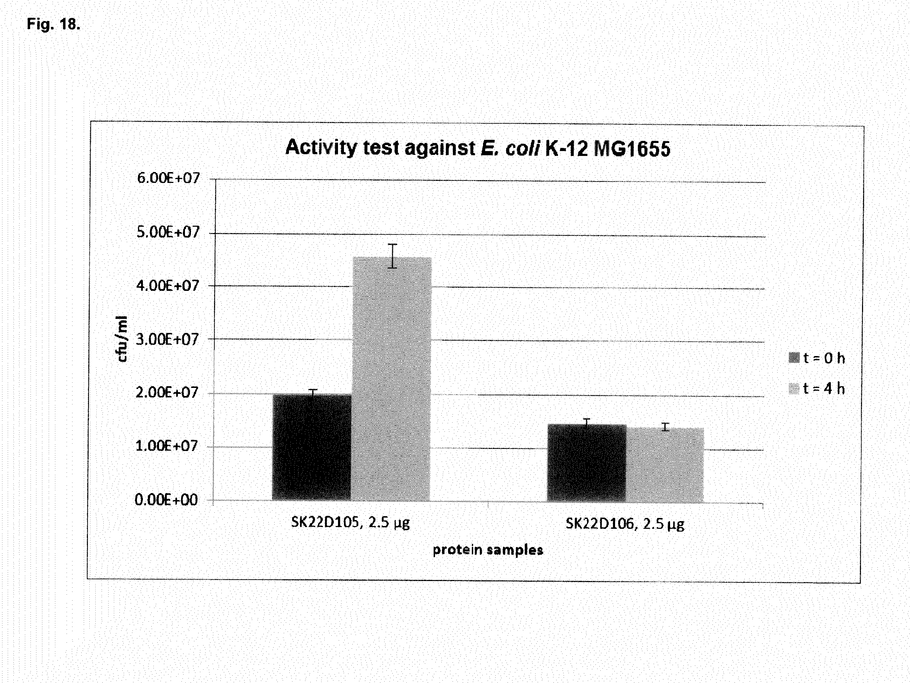

Fig. 18. The antimicrobial activity of secreted YebFMHBD2 against *E. coli* K-12 MG1655 as determined by the killing assay in liquid medium. Triple experiments were carried out and the mean values and standard deviations are presented.

### EXAMPLES

### Material and Methods

### Bacterial strains

*Escherichia coli* strain DH5α (F'Phi80dlacZ DeltaM15 Delta(lacZYA-argF) U169 deoR recA1 endA1 hsdR17 (rk-, mk+) phoA supE44 thi-1 gyrA96 relA1)) was used as the host for gene manipulation. *E. coli* Nissle 1917 (EcN) and EcNc (EcN cured from both its cryptic plasmids) and SK22D (EcN's isogenic microcin-negative mutant) were used for heterogeneous gene expression. All strains used and/or constructed are listed in Table 1.

**Table 1**

| Strains used/constructed hereni | |
|---|---|
| Strains | Characteristics |
| *E. coli* K-12 DH5α | F'Phi80dlacZ DeltaM15 Delta(lacZYA-argF) U169 deoR recA1 endA1 hsdR17 (rk-, mk+) phoA supE44 thi-1 gyrA96 relA1 |
| EcN1219 | ApR, EcN harbouring plasmid pAR1219 |
| EcNc1219 | ApR, EcNc (EcN cured from both cryptic plasmids) harbouring plasmid pAR1219 |
| EcN100 | ApR, KmR, EcN harbouring plasmids pAR1219 and pET-28a(+) |
| EcNc100 | ApR, KmR, EcNc harbouring plasmids pAR1219 and pET-28a(+) |
| EcN101 | ApR, KmR, EcN harbouring plasmids pAR1219 and PEAS101 |
| EcN102 | ApR, KmR, EcN harbouring plasmids pAR1219 and pEAS102 |
| EcN103 | ApR, KmR, EcN harbouring plasmids pAR1219 and pEAS103 |
| EcNc 103 | ApR, KmR, EcNc harbouring plasmids pAR1219 and pEAS103 |
| EcN104 | ApR, KmR, EcN harbouring plasmids pAR1219 and pEAS104 |
| EcN105 | ApR, KmR, EcN harbouring plasm ids pAR1219 and pEAS105 |
| EcN106 | ApR, KmR, EcN harbouring plasmids pAR1219 and pEAS106 |
| SK22D105 | ApR, KmR, SK22D (EcN's isogenic microcin-negative mutant) harbouring plasmids pAR1219 and pEAS105 |
| SK22D106 | ApR, KmR, SK22D harbouring plasmids pAR1219 and pEAS106 |

### Plasmids and enzymes

Plasmid vector pET-28a(+) (Novagen, Madison, Wisconsin, USA) was used for cloning and expression of the target defensin genes. It encodes an N-terminal His-tag and an optional C-terminal His-tag and allows for expression of proteins under the control of the T7 RNA polymerase promoter.

The pBR322-based plasmid pAR1219 (Sigma-Aldrich, Saint Louis, Missouri, USA) encodes the T7-RNA polymerase under the control of the IPTG-inducible lac UV5 promoter (Davanloo et al., 1984). In this study, EcN, EcNc and SK22D were transformed with pAR1219 and a recombinant pET-28a(+) plasmid encoding a defensin.

Moreover, plasmid pYebF01 containing the yebF gene (NCBI (B1847)) under the control of the acrB promoter was constructed.

All restriction enzymes and T4 DNA ligase were purchased from New England BioLabs (NEB, Ipswich, Massachusetts, USA).

Design of HBD2 and HD5 genes with optimized codon usage for expression in *Escherichia coli*

The sequences of the human beta-defensin 2 gene (GeneBank accession No AF040153) and the human alpha-defensin 5 gene (GeneBank accession No M97925) were optimized for high expression in *E. coli* according to the codon usage table (http://www.kazusa.or.jp/codon/). Optimized nHBD2 and nHD5 genes were produced by Eurofins MWG Operon (Ebersberg, Germany) (nHBD2) and Sloning Biotechnology (Munich, Germany) (nHD5), respectively. All primers designed for and used are listed in Table 2.

**Table 2**

| Primers used for amplification and subsequent cloning into vector pET-28a(+) | |
|---|---|
| Primer | Sequence |
| P1 | 5'- CTTTAAGAATTCATGCGTGTT -3' (SEQ ID NO:4) |
| P2 | 5'- CTTATTAAGCTTTCATTACGGT -3' (SEQ ID NO:5) |
| P3 | 5'- CTGCCGGGTGAATTCGGTG -3' (SEQ ID NO:6) |
| P4 | 5'- TCAGGAGGCCGGATCCATG -3' (SEQ ID NO:7) |
| P5 | 5'- GCAGGAATTCGATATCAAGCTT -3' (SEQ ID NO: 8) |
| P6 | 5'- GTACCAGCGGATCCCAGG -3' (SEQ ID NO: 9) |
| P7 | 5'- TAGAGAGCTCCCATGGAGAA -3' (SEQ ID NO: 10) |
| P8 | 5'-GCGTTAATGAGATATCCTCGAGA -3' (SEQ ID NO: 11) |

The underlined part of each primer sequence corresponds to the recognition site of a restriction enzyme: P 1 (EcoRI), P2 (HindIII), P3 (EcoRI), P4 (BamHI), P5 (HindIII), P6 (BamHI), P7 (NcoI), P8 (XhoI).

### Construction of recombinant plasmids encoding defensins

All plasmids used and generated in this application are listed in Table 3. For amplification of the desired DNA fragment, Primers P1 (harboring an EcoRI site) and P2 (harboring a HindIII site) were used for amplification of full-length nHBD2 gene with synthesized nHBD2 gene (by Eurofins MWG Operon) as template and subsequent ligation with EcoRI/HindIII-cut pET-28a(+). The resulting plasmid pEAS101 encodes the fusion protein consisting of HBD2 with an N-terminal extension harboring a His-tag. Similarly, amplification and cloning of the nHBD2-gene-part encoding mature HBD2 with primers P2 and P3 (Table 2) were employed for creating pEAS102. This plasmid encodes mature HBD2 with a His-tag in the N-terminal extension.

In the same way, plasmids pEAS103 encoding the proform of HD5 and pEAS 104 encoding mature HD5, both with an N-terminal His-tag, were constructed using primers P4, P5 and P6 (Table 2).

The construction of the recombinant plasmids started with the amplification of the desired DNA fragment, agarose gel electrophoresis and elution of this fragment using QIAquick Gel Extraction Kit (QIAGEN, Hilden, Germany). Afterwards, the eluted PCR products and pET-28a(+) were digested with EcoRI and HindIII for construction of PEAS101 and pEAS102 and with BamHI and HindIII for cloning of pEAS103 and pEAS104. Each designated digestion product was purified with QIAquick PCR purification Kit (QIAGEN) again. Finally, the insert and pET-28a(+) were ligated using T4 ligase (NEB).

Moreover, a recombinant plasmid was constructed for secretion of mature HBD2. For the new construct, the nMHBD2 gene was amplified using primers P2 and P3 and ligated to EcoRI- and HindIII-cleaved plasmid pYebF01 for fusing it to the carboxyl end of the yebF gene (Table 2). After the insert was confirmed by restriction enzyme analysis, the preferred DNA-sequence was identified. Afterwards, the yebFnMHBD2 gene was cloned into NcoI/HindIII-digested plasmid pET-28a(+), using primers P2 and P7 in order to put it under the control of the T7 RNA polymerase promoter. Moreover, pEAS105 encoding YebF was constructed by using primers P7 and P8.

*E. coli* K-12 DH5α was transformed with the resulting plasmid by electroporation. The colonies obtained were checked for containing the favored insert with restriction enzyme analysis and the correct DNA-sequence was determined. EcN, EcNc and SK22D strains harboring plasmid pAR1219 were subsequently transformed with these recombinant plasmids.

**Table 3**

| Plasmids used in this study | |
|---|---|
| Plasmids | Characteristics |
| pAR1219 | ApR, pBR322 derived plasmid encoding the T7 polymerase under the UV5 promoter control |
| pYebF01 | ApR, Plasmid containing yebF gene under the control of the acrB promoter |
| pET-28a(+) | KmR, Plasmid vector for cloning genes under the expression control of the T7 promoter and for creating a fusion protein containing either an N- or a C-terminal His-tag. |
| pEAS101 | KmR, pET-28a(+) containing nHBD2 gene under the control of the T7 promoter and encoding HisHBD2 fusion protein with an N-terminal His-tag |
| pEAS102 | KmR, pET-28a(+) containing nMHBD2 gene under the control of the T7 promoter and encoding HisMHBD2 fusion protein with an N-terminal Histag |
| pEAS103 | KmR, pET-28a(+) containing nHD5 gene under the control of the T7 promoter and encoding HisHD5 fusion protein with an N-terminal His-tag |
| pEAS104 | KmR, pET-28a(+) containing nMHD5 gene under the control of the T7 promoter and encoding HisMHD5 fusion protein with an N-terminal Histag |
| pEAS105 | KmR, pET-28a(+) containing yebF gene under the control of the T7 promoter and encoding YebF protein |
| pEAS106 | KmR, pET-28a(+) containing yebF and nMHBD2 genes and under the control of the T7 promoter and encoding YebFMHBD2 fusion protein |

### Expression of recombinant defensins with the T7 expression system

Ten microliters of glycerol culture of each recombinant strain (see table 1) were added to 10 ml of Turbo medium (Tryptone, yeast extract, NaCl, glycerol, KH₂PO₄, K₂HPO₄), supplemented with ampicillin (20 µg/ml), kanamycin (10 µg/ml) and protease inhibitor cocktail according to the instructions of the manufacturer (Roche, Basel, Switzerland). Cultures were grown at 37 °C for 16 h. Subsequently, 8 ml of each culture were used to inoculate 800 ml Turbo medium containing ampicillin, kanamycin, and an appropriate amount of protease inhibitor cocktail, as stated above. The cultures were incubated at 37 °C and expression was induced at an OD600 of 1.0 with IPTG (final concentration 1.0 mM) at 37 °C or 20 °C for varying times. Negative controls were always included and treated under the same conditions as the recombinant EcN strains expressing defensins.

### SDS-tricine western blot analysis

Total proteins from whole cells were prepared by centrifugation of induced cultures at 13,000 x g for 10 min, and cells were resuspended in 10 mM Tris-HCl, pH 7.4. Proteins in the cell-free medium were obtained by centrifugation of 2 ml induced culture at 13,000 x g for 10 min and sterile filtration (0.22 µm, Millipore, Billerica, Massachusetts, USA). Afterwards, the supernatant was precipitated with 10 % trichloroacetic acid (TCA) at 4 °C for 1 h. After centrifugation at 13,000 x g and 4 °C for 15 min, the pellet was washed twice by addition of 1 ml 100 % ethanol and once by 1 ml of 70 % ethanol. The final pellet was dissolved in 10 mM Tris-HCl, pH 7.4, SDS-PAGE sample buffer (12 % SDS, 6 % mercaptoethanol, 30 % glycerol, 0.05 % Coomassie blue G-250 (Roth, Karlsruhe, Germany), 150 mM Tris-HCl, pH 7.0) was added at an ¼ volume of each sample, and then samples were boiled at 95 °C for 5 min. SDS-tricine PAGE (12 % acrylamide) was used to check the expression of the recombinant defensin peptides under reducing conditions (Schagger, 2006). After electrophoresis, proteins were transferred to an Immobilon PS-Q membrane (Millipore) in Western blot buffers (Anode buffer I: 0.3 M Tris, 20 % methanol, 0.1 % Tween 20; Anode buffer II: 25 mM Tris, 20 % methanol; Cathode buffer: 25 mM Tris, 20 % methanol, 40 mM ε-Amino-n-capronic acid) with a semi-dry blotting apparatus at 0.8 mA/cm2 for 1 h. The membrane was fixed with 0.01 % glutaraldehyde in Tris-Buffered Saline (TBS) for 20 min, blocked in 5 % non-fat milk for 18 h and probed with a corresponding antibody. Anti-HBD2 rabbit polyclonal antiserum (1:2,000, Tomas Ganz, UCLA, Los Angeles, USA), anti-HD5 rabbit polyclonal antiserum (1:1,000, Edith Porter, California State University, Los Angeles, USA), anti-ß-glactosidase monoclonal antibody (1:1,000, Cell signaling, Beverly, Massachusetts, USA) and anti-maltose-binding protein rabbit polyclonal antiserum (1:5,000, NEB) were used in this work. The blots were processed for chemiluminescent detection (0.1 M Tris pH 8.5, 0.2 mM coumaric acid, 1.25 mM Luminol and 0.06 % H₂O₂).

### Isolation of soluble mature fusion HBD2 with an N-terminal His-tag (HisMHBD2) by nickel-affinity chromatography

The induced cells were collected by centrifugation at 6,000 x g for 20 min. The cells were lysed in LEW buffer (50 mM NaH₂PO₄ 300 mM NaCl, pH 7.8) by sonication (15-sec bursts, 10 times, with a 15-sec cooling period between each burst). Lysed extract was separated from cell debris by centrifugation at 10,000 x g for 30 min, and then the supernatant was filtrated (0.22 µm, Millipore). The filtrate contained the soluble proteins and peptides. To yield the insoluble proteins, the pellet was further dissolved in 8 M urea. The respective defensin peptide can be enriched by nickel-affinity chromatography due to a His-tag. Protino Ni-TED 2000-packed columns (Macherey-Nagel, Düren, Germany) were used for the affinity chromatographic purification of the defensin peptide. A column was equilibrated with 8 ml of LEW buffer and then allowed to drain by gravitation. The soluble fraction of cellular proteins was subjected to nickel-affinity chromatography and the His-tagged peptide was eluted with 9 ml of elution buffer (50 mM NaH₂PO₄, 300 mM NaCl, 250 mM imidazole, pH 7.8). Desalination was performed by dialysis with H₂O at 4 °C for 9 h. During this time, water was changed two times and then samples were concentrated with Spectra Absorbent (Spectrum Laboratories, Inc., Rancho Domingouez, California, USA). The Bradford protein assay was used for subsequent protein quantification (Bradford, 1976).

### Antimicrobial activity tests

Cellular soluble HisMHBD2 was prepared by isolation via nickel-affinity chromatography for activity tests as explained above. For preparation of the secreted YebF-proteins after induction at 37 °C for 4 h, cells were removed by centrifugation at 6,000 x g for 20 min and the supernatant was sterile filtrated (0.22 µm, Millipore) in order to obtain the secreted proteins in the cell-free supernatant. Afterwards, dialysis, concentration, and protein quantification were performed as described before.

Antimicrobial activity of cellular soluble HisMHBD2 and secreted YebFMHBD2 was tested by radial diffusion assays (Lehrer et al., 1991) with *E. coli* K-12 MG1655, *Salmonella enterica* serovar Typhimurium SL1344 and *Listeria monocytogenes* EGD as indicator strains. For radial diffusion assays, bacteria were grown at 37 °C for 18 h in 5 ml of TSB broth (Difco, Lawrence, Kansas, USA). To obtain mid-logarithmic phase bacteria, 5 µl *(E. coli* K-12 MG1655 and *Salmonella enterica* serovar Typhimurium SL1344) or 50 µl *(Listeria monocytogenes)* of the overnight cultures were used to inoculate 5 ml fresh TSB and incubated for an additional 2.5 h at 37 °C. The bacteria were centrifuged at 900 x g and 4 °C for 10 min, washed once with cold 10 mM sodium phosphate buffer, pH 7.4, and resuspended in 2.5 ml of cold 10 mM sodium phosphate buffer. An aliquot containing 4x10⁶ bacterial cells was added to 10 ml of previously autoclaved and warm (42 °C) 10 mM sodium phosphate buffer containing 1 % TSB medium and 1 % low-electroendosmosis-type agarose. The agar was poured into a petri dish and allowed to solidify. Thereafter, a sterile glass pipette (5 mm diameter) was used to create wells in the agar. After adding samples to each well, the plates were incubated at 37 °C for 4 h and then were overlaid with 10 ml of sterile agar, maintained in liquid state by keeping at 42 °C. The overlay agar consisted of a double-strength (6 % w/v) solution of TSB and 1 % agarose. After incubation at 37 °C for 18 h, the inhibition zones surrounding the wells were detected.

Moreover, bioactivity of secreted mature fusion HBD2 was tested in liquid culture. In detail, 2.5 µg of total proteins from the cell-free supernatant were added to log-phase *E. coli* K-12 MG1655 (10⁷ cfu/ml). Each test mixture had a volume of 300 µl and contained secreted proteins/peptides and bacteria in 10 mM sodium phosphate buffer, pH 7.4, and 1 % TSB medium. After mixing all components in the samples, a 100 µl aliquot of each sample was withdrawn for analysis of initial values (time point zero). The residual 200 µl of the samples were incubated at 37 °C for 4 h. All samples were serially diluted and aliquots of 100 µl were spread on LB agar plates. The plates were incubated at 37 °C for 18 h and then the number of colonies was counted.

### RESULTS

### Codon optimization of human β-defensin 2 and human α-defensin 5 genes for expression in Escherichia coli

Differences in codon usage between species can affect the quantity and quality of the proteins expressed by recombinant techniques. Therefore, codons in the cDNA of human β-defensin 2 (HBD2) and human α-defensin 5 (HD5) were analyzed for rarely occurring codons.

HBD2 (GeneBank accession No AF040153) consists of 64 amino acids, 27 amino acids (42.2 %) of which are encoded by rarely used codons in *E. coli.* Moreover, 6 codons (coding for R2, I13, I25, I37, R45 and R46) play important roles in adverse effects on heterogeneous protein expression in *E. coli* (Kane, 1995). Particularly, R2, R45 and R46 are encoded by the least used codons (AGG and AGA) among the minor codons in *E. coli* (Fig. 1). The presence of single AGG or AGA and tandem repeats of them were discovered to dramatically reduce the maximum level of protein synthesis and may also cause frameshifts with high frequency (Spanjaard et al., 1990).

HD5 (GeneBank accession No M97925) consists of 94 amino acids with 29 amino acids (30.9 %) encoded by low-usage codons in *E. coli.* Among these 29 amino acids, 6 amino acids are encoded by codons (coding for R2, R25, R55, R62, R68 and R90) which may cause adverse effects on protein expression in *E. coli* (Fig. 1).

In order to avoid the potential problems of these rare codons on HBD2 and HD5 expression in *E. coli,* corresponding new genes, nHBD2 (EMBL HE583189) and nHD5 (EMBL HE583188) (Fig. 1), were designed where the rarely used codons were replaced by frequently used ones, according to the codon usage table of *E. coli* (http://www.kazusa.or.jp/codon/). To increase the translation termination signal in *E. coli,* the original stop codon TGA was replaced by two stop codons in series, TAATGA, which is the most efficient translational termination sequence in *E. coli* (Hannig and Makrides, 1998).

### Construction of recombinant plasmids for the expression of defensins

Plasmid vector pET-28a(+) (Novagen) (Fig. 2), which encodes two His-tags and allows expression of genes under the control of the T7 promoter was used for the construction of recombinant plasmids coding for fusion proteins consisting of an N-terminal His-tag and a defensin. The synthetic full-length nHBD2-gene or that sequence of the nHBD2-gene encoding the mature part of HBD2 (nMHBD2: amino acids G24 to P64, Fig. 1A) was ligated with the cleaved pET-28a(+) to construct the expression plasmids pEAS101 and pEAS102, respectively (Figs. 3, 4). Plasmid pEAS101 harbors the nHBD2-and pEAS102 encodes the nMHBD2-gene. Both genes were under the control of the T7 promoter and each of the resulting defensins (HisHBD, HisMHBD2) contained an N-terminal His-tag. Plasmids pEAS103 and pEAS104 were constructed in order to express both the proform of HD5 and the mature part of HD5 (nMHD5: amino acids A63 to R94, Fig. 1B). Again, the resulting defensins contained an N-terminal His-tag. The gene product encoded by pEAS103 was HisHD5, and the one encoded by pEAS104 was HisMHD5 (Figs. 5, 6).

### Expression of HBD2 and HD5 fusion proteins in Escherichia coli

Western blot analysis of whole cellular proteins of the induced recombinant strains EcN100, EcN101, and EcN102 was performed with anti-HBD2 rabbit polyclonal antiserum, for detection of HisHBD2 and HisMHBD2 expression. After cell lysis, also soluble and insoluble protein fractions were screened by Western blot analysis with HBD2 rabbit polyclonal antiserum. The negative control, EcN100, was always treated like EcN101 and EcN102, with respect to growth, induction, sample preparation, SDS-Page and Western blot.

HisHBD2 expression was assayed at several time points after induction at 37 °C, because in the clinical setting, after oral administration of the defensin-producing EcN strain, defensin expression should take place in the human gut at the physiological body temperature. However, HisHBD2 was not detected after induction at 37 °C at any time (data not shown). HisHBD2 was observed after induction at 20 °C for 6 h and present in the insoluble pellet of whole cell proteins (Fig. 7). In contrast, after induction of HisMHBD2 expression at 37 °C for 1 h and 2 h, HisMHBD2 was detected in the supernatant (soluble protein fraction) as well as in the pellet (insoluble protein fraction) after cell lysis (Fig. 8). No defensin peptide was detected in corresponding samples of the negative control (EcN100).

Expression of HisHD5 and HisMHD5 in EcN103 and EcN104 was screened by Western blot with HD5-specific rabbit polyclonal antiserum of whole cell proteins and soluble as well as insoluble protein fractions.

HisHD5 was detected after induction at 37 °C for 4 h, however, it was present in the insoluble fraction of total cellular proteins (Fig. 9). HisMHD5 was detected as well, but only a very low amount of HisMHD5 was produced after induction at 37 °C for 1 h and 2 h (Fig. 10). HisMHD55 was not detectable at all after induction at 37 °C for more than 2 h in the presence of the protease inhibitor cocktail (data not shown).

### Isolation by nickel-affinity chromatography and antimicrobial activity of HisMHBD2

HisMHBD2 fusion protein expressed in EcN 103 was eluted from Ni-column and subsequently dialysis and protein quantification were performed as described in Materials and Methods. The resulting material was used for antimicrobial activity tests. Ni-column eluates from EcN100 samples (negative controls) were treated like eluates from HisMHBD2 samples. Antimicrobial activity of eluted proteins/peptides was evaluated by radial diffusion assays employing E. *coli* K-12 MG1655, *Salmonella enterica* serovar Typhimurium SL1344 and *Listeria monocytogenes* EGD as indicator strains.

Inhibition zones could be detected with eluted proteins from strain EcN102 encoding HisMHBD2 (the positive control), while the negative control (eluted proteins from EcN100) did not show any antimicrobial effect (Fig. 11).

Insoluble HisHBD2 from EcN101 and HisHD5 encoded by EcN103 were examined for bioactivity after the refolding procedure using "Protein Refolding kit" (Novagen), however, these protein fractions did not show any activity. HisMHD5 from EcN104 was expressed at a very low yield. Therefore, it was not possible to obtain enough material to get a solution of comparable concentration for activity tests.

### Secretion of a MHBD2 fusion protein by recombinant E. coli strains

*E. coli* protein YebF (10.8 kDa) is a soluble endogenous protein secreted into the medium and it is used as a carrier for transgenic proteins (Zhang et al., 2006). It has been shown that passenger proteins linked to the carboxyl end of YebF are efficiently secreted by the respective recombinant *E. coli* K-12 strain. Therefore, the inventors decided to design a fusion protein consisting of the mature part of HBD2 fused to the C-terminus of YebF in order to achieve secretion of the resulting fusion protein YebFMHBD2 by the respective recombinant *E. coli* strains (Figs. 12, 14). EcN105 harboring plasmids pAR1219 and pEAS105 encoding only yebF (NCBI (B1847)) was used as the negative control and was treated as the recombinant strain encoding a defensin gene (Fig. 13).

Secretion of YebFMHBD2 was tested by analysis of whole cellular proteins and cell-free supernatant samples of induced EcN105 and EcN106 by Western blot. Since the presence of the fusion protein in the culture medium could be a result of the achieved secretion via YebF, cell lysis, leakage through the outer membrane, a natural cell secretion process, or some combination thereof, the inventors analyzed cells and growth medium by Western blot analysis, not only with anti-HBD2 rabbit polyclonal antiserum, but also with a monoclonal antibody specific for the cytoplasmic ß-galactosidase and with a rabbit polyclonal antiserum specific for the periplasmic maltose-binding protein (MBP) (Fig. 15).

YebFMHBD2 was found in the cell-free supernatant after centrifugation and sterile filtration of induced EcN106 cultures, in contrast to EcN105 cultures. Fig. 15 shows the accumulation of YebFMHBD2 in the medium during induction. In the cell-free supernatant, YebFMHBD2 was clearly detected at 2 h and 4 h after induction. After induction for 20 h, most of YebFMHBD2 was present in the medium. In samples representing the bacterial cells, ß-galactosidase, MBP and HBD2 were readily detected. ß-galactosidase, however, was not observed in the medium at all at any time point analyzed. MBP was not detected in the medium at 1.5 h after induction. A trace amount of MBP was detected in the medium at 3 h and 6 h after induction, which indicates the beginning of some inevitable cell lysis.

Moreover, the location of synthesized YebFMHBD2 from EcN106 was compared with the location of HisMHBD2 from EcN102 by Western blot analysis (Fig. 16). YebFMHBD2 was detected in bacterial cells and in the culture medium, whereas HisMHBD2 was present only in the fraction representing total cellular proteins and not in the cell-free supernatant (i.e. culture medium). The fusion defensins were observed to migrate slower than expected from their theoretical molecular mass (HisMHBD2: 8.2 kDa, YebFMHBD2: 17.3 kDa, secreted YebFMHBD2: 15.1 kDa) in Western blot analysis (Fig. 16). Small peptides often fail to follow the standard relationship between mass and mobility in SDS-PAGE (Faurschou et al., 2005; Huang and Matthews, 1990; Weber et al., 1972). Moreover, secreted YebFMHBD2 from the spent culture medium (Fig. 16. Lane 6) was detected as a smaller protein than YebFMHBD2 from the cellular protein pool (Fig. 16. Lane 5), because YebF is cleaved after the 21-amino acid sec-leader (Zhang et al., 2006) during the secretion process. These results demonstrate YebFMHBD2 to be secreted into the medium and not just being released by cell lysis.

### Antimicrobial activity of the secreted YebFMHBD2 fusion protein

Firstly, YebFMHBD2 encoded by EcN106 was tested for antimicrobial activity by radial diffusion assays. However, inhibition zones were already observed with the negative control, secreted proteins from EcN105. The reason could be that EcN produces and secretes the two microcins M and H47 (Patzer et al., 2003).

Therefore, to test for antimicrobial activity of the secreted YebFMHBD2 protein, supernatants of the microcin-negative isogenic SK22D strain harboring pAR1219 and pEAS106 were employed. Cell-free supernatants of induced cultures from SK22D105 and SK22D106 were obtained by centrifugation and sterile filtration. Before antimicrobial tests by radial diffusion assays and killing assays in liquid medium were performed, the respective cell-free supernatant was dialyzed and concentrated.

Only supernatant from SK22D106 showed killing of *E. coli* K-12 MG1655, *Salmonella enterica* serovar Typhimurium SL1344, and *Listeria monocytogenes* EGD in radial diffusion assays (Fig. 17). The same supernatant caused growth inhibition of *E. coli* K-12 MG1655 in liquid culture (Fig. 18). Since the killing assay in liquid culture was performed in the presence of only 1 % TSB medium , *E. coli* K-12 MG1655 growth was also reduced in the presence of the negative control (secreted proteins from SK22D105) after 4 h incubation.

### DISCUSSION

This invention discloses a microbial delivery vector for defensins using a well-known probiotic *E. coli* strain, *E. coli* Nissle 1917 (EcN) (Sonnenborn and Schulze, 2009). The inventors have chosen EcN because this probiotic has an excellent biosafety profile and is used in medical practice since 1917. Moreover, it is marketed as a licensed drug in 8 European countries as well as overseas (in Iran, Lebanon, Peru and South Korea).

To achieve expression of small cationic peptides with antibacterial activity in bacteria, two major barriers must be overcome. These include the potential ability of the cationic peptides to kill the producing strain and the susceptibility of the cationic peptides to proteolytic degradation. Piers and coworkers found that they could overcome such barriers by using a fusion protein expression system (Piers et al., 1993). Therefore, the inventors made use of the low copy number vector, pET-28a(+) expression vector, for cloning defensin genes. The defensin gene was ligated in this vector in a way that gives rise to a fusion gene encoding a protein consisting of an N-terminal peptide harboring a His-tag and the defensin. In addition, the cloning of the respective defensin gene was performed in a way that allowed the expression of the fusion gene under the control of the phi10 T7-polymerase-specific promoter. Expression was detectable after induction of T7 DNA-dependent RNA-polymerase synthesis. The T7 polymerase gene, the expression of which is IPTG-inducible, was provided by plasmid pAR1219. Both plasmids, pAR1219 and the recombinant pET-28a(+), were introduced into EcN. To reduce the risk of killing the producer strain of the His-tagged defensin, induction of expression was performed in Turbo medium containing 86 mM NaCl. As reported by Xu and colleagues (Xu et al., 2006), this concentration of NaCl led to the survival of 80 % of an *E. coli* D31 culture producing HBD2, in contrast to only 40 % survival in a culture without NaCl. In our hands, these measures helped the recombinant EcN strains to grow even during expression of the recombinant defensins.

Another potential problem, defensin degradation, was avoided by adding a protease inhibitor cocktail during incubation. A further approach to impede protein degradation in the absence of a protease inhibitor cocktail is the deletion of genes encoding proteases such as OmpT and Lon in EcN to construct a suitable strain for protein expression comparable to *E. coli* K-12 strains BL21 (DE3) and KRX (Derbise et al., 2003). This will be part of the ongoing work to optimize defensin production by EcN.

In this study, the first defensin to be cloned and produced in EcN was human α-defensin 5 (HD5), which together with human α-defensin 6 is specifically reduced in small intestinal Crohn's disease (Wehkamp and Stange, 2010). The inventors have chosen HD5 because it is encoded as an inactive proform and production of the inactive proform of HD5 should not affect viability of EcN. The other defensin selected for production by EcN was HBD2. This defensin is induced in Caco-2 cells by EcN via its flagellin, and its induction in Crohn's colitis is typically impaired (Schlee et al. 2007, Gersemann et al. 2008).

However, efficient expression of defensin genes in *E. coli* is not achieved with the original DNA sequence, because of different codon usage in humans and in *E. coli.* Therefore, the defensin genes to be cloned were optimized for expression in *E. coli.* It was shown that codon optimization can significantly improve the expression level. About nine-fold improvement of cellular soluble HBD2 expression was observed by optimizing the HBD2 gene with *E. coli*-preferred codons, as reported by Peng and co-workers (Peng et al., 2004). Therefore, two new sequences encoding human β-defensin 2 (nHBD2) and human α-defensin 5 (nHD5) were synthesized with the codon-preference of *E. coli.* Western blots demonstrated successful expression of HBD2 and HD5 as fusion proteins with a His-tag at the N-terminus in probiotic EcN under the control of the T7 promoter. The recombinant EcN strains harbored the recombinant plasmid pET-28a(+) and the T7-polymerase encoding plasmid pAR1219. The full-length HBD2 fusion protein (HisHBD2) was mainly present in the insoluble protein fraction, but the fusion protein containing the mature part of HBD2 fusion protein (HisMHBD2) was expressed in the soluble and insoluble protein fractions. This is in accordance with an earlier study showing mature HBD2 without signal peptide to have a higher solubility than full-length HBD2 (Xu et al., 2006). This is the consequence of the presence of the signal peptide being rich in hydrophobic amino acids (78.3%) in comparison with the mature part of HBD2 (34.1%). The high hydrophobicity of the signal peptide resulted in lower solubility of the HisHBD2 protein, because the exposure of the hydrophobic part might induce aggregation. The signal peptide might also reduce the folding rate or accuracy of disulfide bonds in that part of the molecule representing mature HBD2.

The proform of the HD5 fusion protein (HisHD5), consisting, besides the part of the fusion partner with the His tag of the HD5 signal sequence, of the propiece and of the piece representing the mature HD5, was found only in the insoluble protein fraction without showing bioactivity. Only very low amounts of the fusion protein with the mature HD5 (HisMHD5) could be detected in EcN. Since the signal peptide of HD5 also contains a high percentage of hydrophobic amino acids (78.9 %), this might be the cause for the insolubility of HisHD5. Production of the recombinant mature forms of α-defensins is considered challenging, since the presence of an anionic pre-piece is believed to be important for the inhibition of cytotoxicity of the cationic peptides (Valore et al., 1996). It was reported that the expression of several human α-defensins (hNP-1, hNP-2, hNP-3, hD-5 and hD-6) in bacteria as mature fusion defensins containing a portion of the tryptophan operon of *E. coli* including a His-tag (111 AA) did not result in soluble proteins, because they were mainly found in inclusion bodies (Pazgier and Lubkowski, 2006). Moreover, α-defensins could be produced only with low yields by engineered bacterial systems, and α-defensins were sensitive to rapid degradation by bacterial proteases (Piers et al., 1993; Valore and Ganz, 1997). Up to now, successful expression of human α-defensins in soluble and active form in *E. coli* has not been reported.

In contrast, in this invention mature fusion HBD2 (HisMHBD2) was produced in a soluble form. It is well-known that soluble recombinant proteins are often correctly folded and are usually bioactive (Sorensen and Mortensen, 2005). Therefore, the heterologous expression in bacteria resulting in a soluble protein is very attractive. In fact, the soluble HisMHBD2 obtained in this invention showed antimicrobial activity in radial diffusion assays against *E. coli* K-12 MG1655, *Salmonella enterica* serovar Typhimurium SL1344 and *Listeria monocytogenes* EGD. According to the antimicrobial mechanism of action of defensins, cationic parts of the peptides are capable of interacting with negatively charged structures of the bacterial membrane and then lead to its permeabilization (Ganz, 1999; Peschel, 2002). HisMHBD2 is cationic containing the fusion partner (theoretical pI 9.50), therefore, even the fusion protein HisMHBD2 seems to be able to interact with the cell membranes of microbes. However, as other modes of defensin action are discussed, the inventors cannot exclude other mechanisms of antimicrobial activity exhibited by HisMHBD2 (Yount et al., 2009).

Although leakage or lysis of EcN bacteria in the human gut might occur and could be sufficient to release HBD2 in significant amounts, the inventors constructed a recombinant EcN strain encoding a fusion protein consisting of YebF and the mature part of HBD2, in order to achieve secretion of the YebFMHBD2 product. The carrier protein YebF was used as the fusion partner since "passenger" proteins linked to the C-terminus of YebF were reported to be efficiently secreted from *E. coli* K-12 strains (Zhang et al., 2006). As expected, secretion of YebFMHBD2 from recombinant EcN strains could be demonstrated, and the inventors could show that the appearance of this fusion protein in the cell-free supernatant was not due to cell lysis at early time points. Occurrence of cell leakage/lysis was checked by Western blots specific for the cytoplasmic ß-galactosidase and the periplasmic maltose-binding protein in samples from bacterial cell extracts and samples from the cell-free culture supernatants. The results obtained with YebF resembled closely those reported in the publication by Zhang and colleagues (Zhang et al., 2006). They compared the release of YebF and maltose-binding protein (MBP). In their study, YebF was present in the culture medium at 1.5 h and 3 h after induction, whereas MBP was not present in the medium at 1.5 h and was hardly detected in the medium at 3 h after induction. After induction for 6 h, MBP was primarily localized in the cellular fraction, although some leakage into the culture medium was observed. In contrast, YebF was almost entirely present in the culture medium at 6 h after induction. The authors believed that these data support YebF to be secreted rather than leaking across the outer membrane. The inventors achieved comparable results for the location of MBP. Moreover, the inventors observed that YebFMHBD2 was present in the culture medium at 2 h and 4 h after induction, and its main amount was present in the medium at 20 h after induction. In addition, the location of cytoplasmic ß-galactosidase protein was analyzed in our study, and this protein was only detected in the cell fraction but not in the medium. Moreover, in comparison to the location of YebFMHBD2, HisMHBD2 was only present in the cells at 2 hrs after induction, whereas YebFMHBD2 was present at this time point in the cells and in the medium as well. These results demonstrated that fusion of MHBD2 to the C-terminus of YebF led to efficient secretion of the fusion protein from the producing bacterial cells.

Zhang and colleagues found that by using YebF, e.g. as YebF-hIL2 (human interleukin-2, 15 kDa, very hydrophobic), YebF-α-amylase (α-amylase, 48 kDa, hydrophilic), and YebF-alkaline phosphatase (94 kDa), YebF could carry these fusion proteins in their active states out of the producing bacterial cell and into the medium (Zhang et al., 2006). These data indicate that YebF has the ability to carry proteins of varying size and hydrophobicity/hydrophilicity from the cytoplasm of *E. coli* cells into the medium. Interestingly, cell-free supernatant samples from bacterial cultures expressing YebFMHBD2 had antimicrobial activity. Such samples, in contrast to control samples of cultures expressing only YebF, inhibited growth of the Gram-negative strains *E. coli* K-12 MG1655 and *Salmonella enterica* serovar Typhimurium SL1344 as well as of the Gram-positive *Listeria monocytogenes* strain EGD. Since YebFMHBD2 is cationic including the fusion partner (theoretical pI 8.93), the fusion protein YebFMHBD2 is assumed to interact with negatively charged structures of the bacterial membrane. Obviously, at least part of the YebFMHBD2 protein becomes folded after secretion in a way that allows the MHBD2-part to execute its antimicrobial activity. It can be speculated that MHBD2 is released from YebFMHBD2 in the human gut by the activity of trypsin, as was shown for the proteolytic activation of the inactive proHD5. Trypsin cleaves after arginine and lysine residues, and two arginine residues are the last two amino acid residues of the YebF-part in YebFMHBD2.

In summary, this invention shows the successful construction of recombinant strains of the probiotic *E. coli* Nissle 1917 which not only were able to produce HD5 and HBD2 defensins but also produced antimicrobially active mature HBD2. Furthermore, a microcin-negative EcN mutant harboring plasmid pEAS106 secreted a fusion protein consisting of YebF and the mature part of HBD2 into the culture medium, and the secreted fusion protein showed growth inhibition of *E. coli, Salmonella* and *Listeria monocytogenes.*

In contrast to earlier reports about defensin production in E. *coli* K-12 strains (Huang et al., 2006; Pazgier and Lubkowski, 2006; Xu et al., 2006), this invention reports for the first time the production of active HBD2 defensin in a probiotic *E. coli* strain (Nissle 1917), which is licensed as a drug for the treatment of patients suffering from e.g. diarrhea or ulcerative colitis. This probiotic strain is a good transient colonizer of the human gut as opposed to *E. coli* K-12 strains which are not able to survive in the human digestive tract. Furthermore, to our knowledge, this is also the first invention showing not just production but also secretion of mature HBD2 by an *E. coli* strain which showed activity against pathogenic *Salmonella* and *Listeria* strains.

These constructs and the results achieved with them laid the foundation for the development of EcN as a delivery vehicle for defensins to patients suffering from a deficiency of such antimicrobial peptides. In Crohn's disease, for example, the induction of defense molecules, such as HBD2, is impaired, in part due to a genetic defect resulting in reduced amounts of HBD2 in the gut (Wehkamp and Stange, 2006). Therefore, targeted delivery of HBD2 by a recombinant EcN-strain producing this defensin locally in the gut after oral administration could have a therapeutic effect in Crohn's disease patients.

### REFERENCES

Bensch K.W., Raida M., Magert H.J., Schulz-Knappe P., Forssmann W.G., 1995. hBD-1: a novel beta-defensin from human plasma. FEBS Lett. 368, 331-335.
Boudeau J., Glasser A.L., Julien S., Colombel J.F., Darfeuille-Michaud A., 2003. Inhibitory effect of probiotic Escherichia coli strain Nissle 1917 on adhesion to and invasion of intestinal epithelial cells by adherent-invasive E. coli strains isolated from patients with Crohn's disease. Aliment. Pharmacol. Ther. 18, 45-56.
Bradford M.M., 1976. A rapid and sensitive method for the quantitation of microgram quantities of protein utilizing the principle of protein-dye binding. Anal. Biochem. 72, 248-254.
Chertov O., Michiel D.F., Xu L., Wang J.M., Tani K., Murphy W.J., Longo D.L., Taub D.D., Oppenheim J.J., 1996. Identification of defensin-1, defensin-2, and CAP37/azurocidin as T-cell chemoattractant proteins released from interleukin-8-stimulated neutrophils. J. Biol. Chem. 271, 2935-2940.
Davanloo P., Rosenberg A.H., Dunn J.J., Studier F.W., 1984. Cloning and expression of the gene for bacteriophage T7 RNA polymerase. Proc. Natl. Acad. Sci. USA 81, 2035-2039.
Derbise A., Lesic B., Dacheux D., Ghigo J.M., Carniel E., 2003. A rapid and simple method for inactivating chromosomal genes in Yersinia. FEMS Immunol. Med. Microbiol. 38, 113-116.
Faurschou M., Kamp S., Cowland J.B., Udby L., Johnsen A.H., Calafat J., Winther H., Borregaard N., 2005. Prodefensins are matrix proteins of specific granules in human neutrophils. J. Leukocyte Bio. 78, 785-793.
Fellermann K., Stange D.E., Schaeffeler E., Schmalzl H., Wehkamp J., Bevins C.L., Reinisch W., Teml A., Schwab M., Lichter P., Radlwimmer B., Stange E.F., 2006. A chromosome 8 gene-cluster polymorphism with low human beta-defensin 2 gene copy number predisposes to Crohn's disease of the colon. Am. J. Hum. Genet. 79, 439-448.
Fric P., Zavoral M., 2003. The effect of non-pathogenic Escherichia coli in symptomatic uncomplicated diverticular disease of the colon. Eur. J. Gastroenterol. Hepatol. 15, 313-315.
Ganz T., 1999. Defensins and host defense. Science 286, 420-421.
Ganz T., Lehrer R.I., 1994. Defensins. Curr. Opin. Immunol. 6, 584-589.
Ganz T., Selsted M.E., Szklarek D., Harwig S.S., Daher K., Bainton D.F., Lehrer R.I., 1985. Defensins. Natural peptide antibiotics of human neutrophils. J. Clin. Invest. 76, 1427-1435.
Garcia J.R., Krause A., Schulz S., Rodriguez-Jimenez F.J., Kluver E., Adermann K., Forssmann U., Frimpong-Boateng A., Bals R., Forssmann W.G., 2001a. Human beta-defensin 4: a novel inducible peptide with a specific salt-sensitive spectrum of antimicrobial activity. FASEB J. 15, 1819-1821.
Garcia J.R., Jaumann F., Schulz S., Krause A., Rodriguez-Jimenez J., Forssmann U., Adermann K., Kluver E., Vogelmeier C., Becker D., Hedrich R., Forssmann W.G., Bals R., 2001 b. Identification of a novel, multifunctional beta-defensin (human beta-defensin 3) with specific antimicrobial activity. Its interaction with plasma membranes of Xenopus oocytes and the induction of macrophage chemoattraction. Cell Tissue Res. 306, 257-264.
Gersemann M., Wehkamp J., Fellermann K. And Stange E.F., 2008. Crohn's disease-defect in innate defence. World J. Gastroenterol., 14, 5499-5503.
Ghosh D., Porter E., Shen B., Lee S.K., Wilk D., Drazba J., Yadav S.P., Crabb J.W., Ganz T., Bevins C.L., 2002. Paneth cell trypsin is the processing enzyme for human defensin-5. Nat. Immunol. 3, 583-590.
Hannig G., Makrides S.C., 1998. Strategies for optimizing heterologous protein expression in Escherichia coli. Trends Biotechnol. 16, 54-60.
Harder J., Bartels J., Christophers E., Schroder J.M., 1997. A peptide antibiotic from human skin. Nature 387, 861.
Harder J., Bartels J., Christophers E., Schroder J.M., 2001. Isolation and characterization of human beta -defensin-3, a novel human inducible peptide antibiotic. J. Biol. Chem. 276, 5707-5713.
Henker J., Laass M., Blokhin B.M., Bolbot Y.K., Maydannik V.G., Elze M., Wolff C., Schulze J.,2007. The probiotic Escherichia coli strain Nissle 1917 (EcN) stops acute diarrhoea in infants and toddlers. Eur. J. Pediatr. 166, 311-318.
Henker J., Laass M.W., Blokhin B.M., Maydannik V.G., Bolbot Y.K., Elze M., Wolff C., Schreiner A., Schulze J., 2008. Probiotic Escherichia coli Nissle 1917 versus placebo for treating diarrhea of greater than 4 days duration in infants and toddlers. Pediatr. Infect. Dis. J. 27, 494-499.
Huang J.M., Matthews H.R., 1990. Application of sodium dodecyl sulfate-gel electrophoresis to low molecular weight polypeptides. Anal. Biochem. 188, 114-117.
Huang L., Wang J., Zhong Z., Peng L., Chen H., Xu Z., Cen P., 2006. Production of bioactive human beta-defensin-3 in Escherichia coli by soluble fusion expression. Biotechnol. Lett. 28, 627-632.
Hugot J.P., Chamaillard M., Zouali H., Lesage S. Cézard J.P., Belaiche J., Almer S., Tysk C., O'Morain C.A., Gassull M., Binder V., Finkel Y., Cortot A., Modigliani R., Laurent-Puig P., Gower-Rousseau C., Macry J., Colombel J.F., Sahbatou M., Thomas G., 2001. Association of NOD2 leucinerich repeat variants with susceptibility to Crohn's disease. Nature 411, 599-603.
Kagan B.L., Selsted M.E., Ganz T., Lehrer R.I., 1990. Antimicrobial defensin peptides form voltage-dependent ion-permeable channels in planar lipid bilayer membranes. Proc. Natl. Acad. Sci. USA 87, 210-214.
Kane J.F., 1995. Effects of rare codon clusters on high-level expression of heterologous proteins in Escherichia coli. Curr. Opin. Biotechnol. 6, 494-500.
Kruis W., Schütz E., Fric P., Fixa B., Judmaier G., Stolte M., 1997. Double-blind comparison of an oral Escherichia coli preparation and mesalazine in maintaining remission of ulcerative colitis. Aliment. Pharmacol. Ther. 11, 853-858.
Kruis W., Fric P., Pokrotnieks J., Lukas M., Fixa B., Kascak M., Kamm M.A., Weismueller J., Beglinger C., Stolte M., Wolff C., Schulze J., 2004. Maintaining remission of ulcerative colitis with the probiotic Escherichia coli Nissle 1917 is as effective as with standard mesalazine. Gut 53, 1617-1623.
LaVallie E.R., Lu Z., Diblasio-Smith E.A., Collins-Racie L.A., McCoy J.M., 2000. Thioredoxin as a fusion partner for production of soluble recombinant proteins in Escherichia coli. Methods Enzymol. 326, 322-340.
Lee J.H., Kim J.H., Hwang S.W., Lee W.J., Yoon H.K., Lee H.S., Hong S.S., 2000. High-level expression of antimicrobial peptide mediated by a fusion partner reinforcing formation of inclusion bodies. Biochem. Biophys. Res. Commun. 277, 575-580.
Lehrer R.I., Lichtenstein A.K., Ganz T., 1993. Defensins: antimicrobial and cytotoxic peptides of mammalian cells. Annu. Rev. Immunol. 11, 105-128.
Lehrer R.I., Rosenman M., Harwig S.S., Jackson R., Eisenhauer P., 1991. Ultrasensitive assays for endogenous antimicrobial polypeptides. J. Immunol. Methods 137, 167-173.
Martin E., Ganz T., Lehrer R.I., 1995. Defensins and other endogenous peptide antibiotics of vertebrates. J. Leukoc. Biol. 58, 128-136.
Matthes H., Krummenerl T., Giensch M., Wolff C., Schulze J., 2010. Clinical trial: probiotic treatment of acute distal ulcerative colitis with rectally administered Escherichia coli Nissle 1917 (EcN). BMC Complement. Alternat. Med. 10, 13.
Nissen-Meyer J., Nes I.F., 1997. Ribosomally synthesized antimicrobial peptides: their function, structure, biogenesis, and mechanism of action. Arch. Microbiol. 167, 67-77.
Ogura Y., Bonen D.K., Inohara N., Nicolae D.L., Chen F.F., Ramos R., Britton H., Moran T., Karaliuskas R., Duerr R.H., Achkar J.P., Brant S.R., Bayless T.M., Kirschner B.S., Hanauer S.B., Nuñez G., Cho J.H. 2001. A frameshift mutation in NOD2 associated with susceptibility to Crohn's disease. Nature 411, 603-606.
Patzer S.I., Baquero M.R., Bravo D., Moreno F., Hantke K., 2003. The colicin G, H and X determinants encode microcins M and H47, which might utilize the catecholate siderophore receptors FepA, Cir, Fiu and IroN. Microbiology. 149, 2557-25570.
Pazgier M., Lubkowski J., 2006. Expression and purification of recombinant human alpha-defensins in Escherichia coli. Protein Expr. Purif. 49, 1-8.
Peng L., Xu Z., Fang X., Wang F., Yang S., Cen P., 2004. Preferential codons enhancing the expression level of human beta-defensin-2 in recombinant Escherichia coli. Protein Pept. Lett. 11, 339-344.
Peschel A., 2002. How do bacteria resist human antimicrobial peptides? Trends Microbiol. 10, 179-186.
Piers K.L., Brown M.H., Hancock R.E., 1993. Recombinant DNA procedures for producing small antimicrobial cationic peptides in bacteria. Gene 134, 7-13.
Porter E.M., Poles M.A., Lee J.S., Naitoh J., Bevins C.L., Ganz T., 1998. Isolation of human intestinal defensins from ileal neobladder urine. FEBS Lett. 434, 272-276.
Raj P.A., Dentino A.R., 2002. Current status of defensins and their role in innate and adaptive immunity. FEMS Microbiol. Lett. 206, 9-18.
Rembacken B.J., Snelling A.M., Hawkey P.M., Chalmers D.M., Axon A.T.R., 1999. Non-pathogenic Escherichia coli versus mesalazine for the treatment of ulcerative colitis: a randomised trial. Lancet 354, 635-639.
Salzman N.H., Ghosh D., Huttner K.M., Paterson Y., Bevins C.L., 2003. Protection against enteric salmonellosis in transgenic mice expressing a human intestinal defensin. Nature 422, 522-526.
Schagger H., 2006. Tricine-SDS-PAGE. Nat. Protoc. 1, 16-22.
Schlee M., Wehkamp J., Altenhoefer A., Oelschlaeger T.A., Stange E.F., Fellermann K., 2007. Induction of human beta-defensin 2 by the probiotic Escherichia coli Nissle 1917 is mediated through flagellin. Infect. Immun. 75, 2399-2407.
Schroder J.M. and Harder J., 1999. Human beta-defensin-2. Int. J. Biochem. Cell. Biol. 31, 645-651.
Schultz M., 2008. Clinical use of E. coli Nissle 1917 in inflammatory bowel disease. Inflamm. Bowel. Dis. 14, 1012-1018.
Shimoda M., Ohki K., Shimamoto Y., Kohashi O., 1995. Morphology of defensin-treated Staphylococcus aureus. Infect. Immun. 63, 2886-2891.
Singh P.K., Jia H.P., Wiles K., Hesselberth J., Liu L., Conway B.A., Greenberg E.P., Valore E.V., Welsh M.J., Ganz T., Tack B.F., McCray P.B. J., 1998. Production of beta-defensins by human airway epithelia. Proc. Natl. Acad. Sci. USA 95, 14961-14966.
Sonnenborn U., Schulze J., 2009. The non-pathogenic Escherichia coli strain Nissle 1917- features of a versatile probiotic. Microb. Ecol. Health Dis. 21, 122-158.
Sorensen H.P., Mortensen K.K., 2005. Soluble expression of recombinant proteins in the cytoplasm of Escherichia coli. Microb. Cell. Fact. 4, 1.
Spanjaard R.A., Chen K., Walker J.R., Van Duin J., 1990. Frameshift suppression at tandem AGA and AGG codons by cloned tRNA genes: assigning a codon to argU tRNA and T4 tRNA(Arg). Nucleic Acids Res. 18, 5031-5036.
Valore E.V., Ganz T., 1997. Laboratory production of antimicrobial peptides in native conformation. Methods Mol. Biol. 78, 115-131.
Valore E.V., Martin E., Harwig S.S., Ganz T., 1996. Intramolecular inhibition of human defensin HNP-1 by its propiece. J. Clin. Invest. 97, 1624-1629.
van Es J.H., Jay P., Gregorieff A., van Gijn M.E., Jonkheer S., Hatzis P., Thiele A., van den Born M., Begthel H., Brabletz T., Taketo M.M., Clevers H., 2005. Wnt signalling induces maturation of Paneth cells in intestinal crypts. Nat. Cell. Biol. 7, 381-386.
Weber K., Pringle J.R., Osborn M., 1972. Measurement of molecular weights by electrophoresis on SDS-acrylamide gel. Methods. Enzymol. 26, 3-27.
Wehkamp J., Stange E.F., 2010. Paneth's disease. J. Crohn. Colitis. 5, 523-531.
Wehkamp J., Wang G., Kübler I., Nuding S., Gregorieff A., Schnabel A., Kays R.J., Fellermann K., Burk O., Schwab M., Clevers H., Bevins C.L., Stange E.F., 2007. The Paneth cell alpha-defensin deficiency of ileal Crohn's disease is linked to Wnt/Tcf-4. J. Immunol. 179, 3109-3118.
Wehkamp J., Stange E.F., 2006. A new look at Crohn's disease: breakdown of the mucosal antibacterial defense. Ann. N.Y. Acad. Sci. 1072,321-331.
Wehkamp J., Salzman NH., Porter E., Nuding S., Weichenthal M., Petras R.E., Shen B., Schaeffeler E., Schwab M., Linzmeier R., Feathers R.W., Chu H., Lima H. Jr., Fellermann K., Ganz T., Stange E.F., Bevins C.L., 2005. Reduced Paneth cell alpha-defensins in ileal Crohn's disease. Proc. Natl. Acad. Sci.U S A 102, 18129-18134.
Wehkamp J., Harder J., Wehkamp K., Wehkamp-von Meissner B., Schlee M., Enders C., Sonnenborn U., Nuding S., Bengmark S., Fellermann K., Schröder J.M., Stange E.F., 2004a. NF-kappaB- and AP-1-mediated induction of human beta defensin-2 in intestinal epithelial cells by Escherichia coli Nissle 1917: a novel effect of a probiotic bacterium. Infect. Immun. 72, 5750-5758.
Wehkamp J., Harder J., Weichenthal M., Schwab M., Schaffeler E., Schlee M., Herrlinger K.R., Stallmach A., Noack F., Fritz P., Schröder J.M., Bevins C.L., Fellermann K., Stange E.F.. 2004b. NOD2 (CARD15) mutations in Crohn's disease are associated with diminished mucosal alpha-defensin expression. Gut 53, 1658-1664.
Xu Z., Peng L., Zhong Z., Fang X., Cen P., 2006. High-level expression of a soluble functional antimicrobial peptide, human beta-defensin 2, in Escherichia coli. Biotechnol. Prog. 22, 382-386.
Yamaguchi Y., Nagase T., Makita R., Fukuhara S., Tomita T., Tominaga T., Kurihara H., Ouchi Y., 2002. Identification of multiple novel epididymis-specific beta-defensin isoforms in humans and mice. J. Immunol, 169, 2516-2523.
Yang D., Chertov O., Bykovskaia S.N., Chen Q., Buffo M.J., Shogan J., Anderson M., Schröder J.M., Wang J.M., Howard O.M., Oppenheim J.J., 1999. Beta-defensins: linking innate and adaptive immunity through dendritic and T cell CCR6. Science 286, 525-528.
Yount N.Y., Kupferwasser D., Spisni A., Dutz S.M., Ramjan Z.H., Sharma S., Waring A.J., Yeaman M.R., 2009. Selective reciprocity in antimicrobial activity versus cytotoxicity of hBD-2 and crotamine. Proc. Natl. Acad. Sci. USA 106, 14972-14977.
Zhang G., Brokx S., Weiner J.H., 2006. Extracellular accumulation of recombinant proteins fused to the carrier protein YebF in Escherichia coli. Nat. Biotechnol. 24, 100-104.

## Claims

1. A recombinant *E. coli* Nissle 1917 (EcN) cell transformed with a nucleic acid coding for a defensin protein or a derivative thereof.

2. The recombinant cell of claim 1, wherein the defensin protein is selected from human α- or β-defensin.

3. The recombinant cell of claim 2, wherein the defensin protein is selected from human α-defensins HNP1-4, human α-defensin 5 (HD5), human α-defensin 6 (HD6), and human β-defensins 1-6 (HBD 1-6), or from the respective mature form of said proteins.

4. The recombinant cell of one or more of claims 1-3, wherein the defensin protein or derivative thereof is expressed as a fusion protein, preferably as a fusion protein with a His-tag or with the *E. coli* YebF protein.

5. The recombinant cell of one or more of claims 1-4, wherein the nucleic acid coding for the defensin protein or derivative thereof is adapted for expression in *E. coli* cells by codon optimization.

6. The recombinant cell of one or more of claims 1-6, wherein the nucleic acid coding for the defensin protein or derivative thereof is selected from SEQ ID NO: 1, 2 or 3.

7. The recombinant cell of one or more of claims 1-5, wherein the nucleic acid coding for the defensin protein or derivative thereof is part of an expression vector, which expression vector additionally contains one or more regulatory sequences.

8. The recombinant cell of claim 7, wherein the expression vector is a plasmid and the regulatory sequences comprise the T7 promoter.

9. A pharmaceutical composition comprising recombinant *E. coli* Nissle 1917 (EcN) cells of one or more of claims 1-8 and a pharmaceutically acceptable carrier.

10. A recombinant *E. coli* Nissle 1917 (EcN) cell of one or more of claims 1-8 for use in the treatment of Crohn's disease.

11. A method of producing a recombinant *E. coli* Nissle 1917 (EcN) cell according to one or more of claims 1-8, comprising the steps of providing a nucleic acid coding for a defensin protein or derivative thereof and *E. coli* Nissle 1917 (EcN) cells, and transforming the cells with said nucleic acid, where the transformed *E. coli* Nissle 1917 (EcN) cells are capable of expressing a defensin protein or derivative thereof.

12. The method of claim 11, wherein the nucleic acid is cloned into an expression vector and introduced into the cell by electroporation.

13. The method of claim 11 or 12, wherein the defensin protein or derivative thereof is expressed as a fusion protein with an N-terminal His-tag.

14. The method of one or more of claims 11 -13, wherein the defensin protein or derivative thereof is expressed as a fusion protein with the *E. coli* YebF protein.
